# FASCICULE DE BREVET EUROPEEN

(11) **EP 0 792 167 B1**
(45) Date de publication et mention de la délivrance du brevet: **27.06.2001**
(21) Numéro de dépôt: 95940329.6
(22) Date de dépôt: 17.11.1995
(51) Int. Cl.: A61K 47/48

(54) **CONJUGUES MONOFONCTIONNELS ET/OU POLYFONCTIONNELS DE LA POLYLYSINE**
MONO- ODER POLYFUNKTIONELLE KONJUGATE VON POLYLYSINE
MONOFUNCTIONAL AND/OR POLYFUNCTIONAL POLYLYSINE CONJUGATES

(30) Priorité: 18.11.1994 FR 9413861
(43) Date de publication de la demande: 03.09.1997
(73) Titulaire: GEFFARD, Michel, F-33400 Talence (FR)
(72) Inventeur: GEFFARD, Michel, F-33400 Talence (FR)
(74) Mandataire: Gillard, Marie-Louise
(86) Numéro de dépôt international: FR9501517
(87) Numéro de publication internationale: WO9615810

(56) Documents cités:
- EP-A- 0 210 412
- WO-A-79/00515
- WO-A-91/13097
- WO-A-92/16221
- WO-A-93/25197
- WO-A-94/27151
- CHEMICAL ABSTRACTS, vol. 121, no. 17, 24 Octobre 1994 Columbus, Ohio, US; abstract no. 195099, CITRO, G. ET AL 'Inhibition of leukemia cell proliferation by folic acid-polylysine-mediated introduction of c-myb antisense oligodeoxynucleotides into HL-60 cells' & BR. J. CANCER (1994), 69(3), 463-7 CODEN: BJCAAI;ISSN: 0007-0920,
- JOURNAL OF IMMUNOLOGICAL METHODS, vol. 15, NEW YORK US, pages 61-67, A. KAMMEYER ET AL. 'PREPARATION OF MONOCLONAL MOUSE ANTIBODIES AGAINST TWO SPECIFIC EU-MELANIN RELATED COMPOUNDS.'
- PROCEEDINGS OF THE NATIONAL ACADEMY OF SCIENCES OF USA, vol. 81, WASHINGTON US, pages 3888-3892, PHILIPPE SEGUELA ET AL. 'ANTIBODIES AGAINST GAMMA-AMINOBUTYRIC ACID: SPECIFICITY STUDIES AND IMMUNOCYTOCHEMICAL RESULTS.' cité dans la demande
- BRAIN RES. (1986), 365(1), 179-84 CODEN: BRREAP;ISSN: 0006-8993, CAMPISTRON, GHISLAINE ET AL 'Specific antibodies against aspartate and their immunocytochemical application in the rat brain'

## Description

La présente invention concerne le domaine médical. Plus particulièrement, l'invention concerne l'utilisation d'au moins un conjugué monofonctionnel et/ou polyfonctionnel de la polylysine pour la préparation de compositions ou associations pharmaceutiques utiles dans le traitement de la dégénérescence neuronale, des neuropathies infectieuses, traumatiques et toxiques, des affections dégénératives à caractère autoimmun, des troubles neurodégénératifs consécutifs à des affections génétiques et des affections prolifératives. L'invention concerne également des conjugués monofonctionnels et polyfonctionnels de la polylysine.

La majorité des affections humaines chroniques invalidantes, telles que la sclérose en plaques (SEP), les polyarthrites (PR), les neuropathies, etc., n'ont pas d'étiologie clairement définie. Par conséquent, les traitements actuellement pratiqués restent symptomatiques utilisation de corticoïdes, d'anti-inflammatoires divers, d'immunodépresseurs, etc.; ces médications présentent de nombreux effets secondaires non négligeables pour la santé des patients. De plus, leur efficacité est fugace. On ne note pas d'amélioration durable et surtout peu d'amélioration dans l'évolution de ces affections.

Des données et travaux récents sur l'auto-immunité (Daverat et al. 1989 (1), Amara et al., 1994 (2)) ont permis d'identifier des cibles antigéniques chimiquement définies par les anticorps circulant dans les fluides biologiques des malades. L'élévation de ces immunoglobulines circulantes est liée à l'évolution de ces maladies. Les anticorps n'apparaissent plus comme des éléments aberrants mais sont le reflet de désordres antigéniques précis.

A titre d'exemple, on a identifié des anticorps dirigés contre l'acide oléique conjugué, l'acide azélaïque conjugué, un phospholipide et le cystéinyl-NO dans la SEP (Maneta-Peyret et al., 1987 (3) ; Daverat et al., 1989 (1) ; Brochet et al., 1991 (4) ; Boullerne et al., 1994 (5)).

De même lors de l'infection par le virus du VIH, des anticorps dirigés contre les acides gras couplés par liaison amide ont été trouvés (Amara et al., 1994 (2)).

La demande WO 94/27151, publiée le 24 novembre 1994, décrit l'utilisation d'au moins un conjugué entre d'une part une molécule susceptible d'être reconnue par:
- les anticorps dirigés contre des acides gras à chaîne linéaire ou ramifiée, saturés ou insaturés, en C₄-C₂₂;
- les anticorps dirigés contre les isoprénoïdes en C₆-C₂₀ liés à une cystéine; et/ou
- les anticorps dirigés contre le cholestérol et ses dérivés,
et, d'autre part, une molécule porteuse d'une taille suffisante pour permettre la reconnaissance de cette molécule par lesdits anticorps, telle que la polylysine, pour la préparation de médicaments destinés au traitement du SIDA.

On a aussi décrit un anticorps anti-choline glutaryl poly-L-lysine, mimant l'acétylcholine (Geffard et al., 1985 (11) ; Souan et al., 1986 (12)), des antisérums R-glutaraldéhyde-polylysine dans lesquels R est un composé indolealkylamine, dirigés contre les indolealkylamines (Geffard et al. 1985 (13)), des anticorps dirigés contre l'acide γ-aminobutyrique (Geffard et al., 1985 (14) ; Seguela et al., 1984 (15)) ainsi qu'un conjugué aspartate-polylysine utilisé dans la caractérisation immunochimique des anticorps polyclonaux anti-aspartate conjugué (Campistron et al., 1986 (21)).

Au cours des affections chroniques citées précédemment, des processus oxydatifs ont été trouvés et décrits (Buttke et al. 1994 (6)). C'est ainsi que dans la SEP, des auteurs ont montré une élévation directe des produits de lipopéroxydation (Hunter et al. 1985 (7), Korpela et al. 1989 (8)) et une diminution notable des anti-oxydants.

Les données récentes montrent qu'au cours de l'infection à VIH, le stress oxydatif serait l'un des éléments déterminants dans la mort cellulaire, donc la perte en lymphocytes TCD4⁺ (Roederer et al. 1993 (9) ; Staal et al. 1992 (10)).

Il était donc indispensable de mettre à la disposition des malades des médicaments ayant le triple objectif suivant :
- restituer les perturbations antigéniques spécifiquement identifiées lors d'une affection humaine chronique ;
- lutter contre les processus inflammatoires, oxydatifs ;
- inhiber les agents étiologiques viraux et/ou bactériens pouvant être à l'origine des affections chroniques évolutives.

Le Demandeur a résolu ce problème en utilisant des conjugués monofonctionnels et/ou polyfonctionnels de la poly-L-lysine à visée thérapeutique.

Selon un premier aspect, la présente invention concerne donc l'utilisation d'au moins un conjugué monofonctionnel et/ou polyfonctionnel de la poly-L-lysine pour la préparation de compositions ou associations pharmaceutiques utiles dans le traitement de la dégénérescence neuronale, des neuropathies infectieuses, traumatiques ou toxiques, des affections dégénératives à caractère autoimmun, des troubles neurodégénératifs consécutifs à des affections génétiques et des affections prolifératives.

La poly-L-lysine (ci-après appelée polylysine) est un polypeptide ou homopolymère de la lysine dont la longueur de chaîne varie suivant la méthode de préparation. Elle répond à la formule: 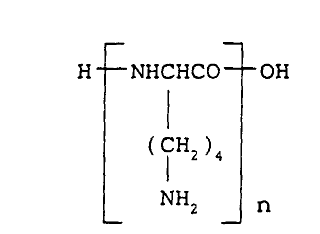 (cf Merck Index, 10ème édition, abrégé N^{•} 7444). Ce produit a un poids moléculaire moyen qui est généralement compris entre 2000 et 300 000, ce qui signifie que son degré de polymérisation (c'est à dire la valeur de l'indice n ci-dessus) est compris entre environ 15 et environ 2345. Bien entendu, par polylysine on entend de la polylysine linéaire ou ramifiée. Avantageusement, on utilise selon la présente invention de la polylysine linéaire possédant un poids moléculaire moyen compris entre 20 000 et 30 000.

Par conjugués monofonctionnels et polyfonctionnels de la polylysine, on entend respectivement les produits de couplage de l'une quelconque des molécules définies ci-après à la polylysine et les produits de couplage d'au moins deux molécules définies ci-après à au moins une molécule de polylysine. Par exemple, un conjugué bifonctionnel peut être représenté par la formule :
molécule 1 -- polylysine -- molécule 2

Avantageusement, les conjugués polyfonctionnels utilisés dans le cadre de l'invention sont des conjugués bifonctionnels.

Les molécules utilisées dans le cadre de l'invention, désignées sous le terme "d'haptène" dans les préparations ci-après, peuvent être choisies dans l'une des trois catégories suivantes :
1/ Les molécules à caractère "acide gras ou lipidique", qui comprennent :
   - les acides gras mono- ou dicarboxyliques, ci-après communémént désignés par le terme (di)acide gras, à chaîne linéaire ou ramifiée, saturés ou insaturés, comprenant généralement de 4 à 24 atomes de carbone ;
   - les composés impliqués dans le mécanisme d'ancrage des protéines aux membranes cellulaires, ces composés intervenant notamment dans le cycle du mévalonate, en particulier les isoprénoïdes liés à une cystéine ;
   - le cholestérol et ses dérivés, notamment les hormones hydrophobes.
2/ Les molécules à caractère "anti-oxydant", qui comprennent :
   - la vitamine A, la vitamine C, la vitamine E ou l'un de leurs dérivés ;
   - la cystéine et ses dérivés, de formule :

      R₁S-R₂-CH(NH₂)-COOH

      dans laquelle R₁ représente H ou CH₃ et R₂ représente un C₁-C₃ alkylène.
3/ Les molécules à caractère "acide aminé ou neurotransmetteur", qui comprennent :
   - les indolealkylamines ;
   - les catécholamines;
   - les acides aminés de formule :

      R₃-CH(NH₂)-COOH

      dans laquelle R₃ représente l'hydrogène, un groupe imidazol-5-ylméthyle, un groupe carboxyméthyle ou un groupe aminopropyle ;
   - les acides amino(C₁-C₅)alkylsulfoniques ou sulfiniques;
   - la carnitine ou la carnosine;
   - les diamines de formule :

      ₂HN-A-NH₂

      dans laquelle A représente un (C₁-C₆)alkylène ou un groupe -(CH₂)ₘ-NH-B-(CH₂)ₚ-dans lequel m et p, indépendamment l'un de l'autre, sont des entiers allant de 1 à 5, et B représente rien ou un groupe -(CH₂)ₙ-NH-, n étant un entier allant de 1 à 5 ;
   - l'acétylcholine et
   - l'acide γ-aminobutyrique.

On distinguera donc trois sortes de conjugués (qu'ils soient monofonctionnels ou polyfonctionnels) : les conjugués "acide gras ou lipidiques"; les conjugués "anti-oxydants"; et les conjugués "acides aminés ou neurotransmetteurs".

Selon une variante de l'invention, on utilise au moins 4 conjugués monofonctionnels et/ou polyfonctionnels de la polylysine pour la préparation de compositions ou associations pharmaceutiques pour le traitement des troubles mentionnés précédemment. Il est possible de n'utiliser que des conjugués de la même sorte. Avantageusement, on utilise des conjugués de deux sortes différentes. De manière préférée, on utilise des conjugués de chacune des trois sortes ci-dessus.

Selon une autre variante de l'invention, il est possible d'utiliser également un ou deux anticorps monoclonaux anti-idiotypiques pour préparer les compositions ou associations pharmaceutiques mentionnées précédemment. Ces anticorps sont par exemple ceux décrits par Chagnaud et al. (18, 19, 20).

Généralement, le (di)acide gras comprend de 4 à 24 atomes de carbone, comme par exemple l'acide butyrique, maléique, succinique, glutarique, adipique, pimélique, subérique, sébacique, caproïque, caprylique, caprique, laurique, myristique, palmitique, palmitoléique, stéarique, oléique, linoléique, γ-linolénique, α-linolénique, arachidique, gadoléique, arachidonique, béthénique, érucique, clupadonique ou azélaïque . De préférence, le (di)acide gras est choisi parmi l'acide myristique, l'acide palmitique, l'acide stéarique, l'acide oléique ou l'acide azélaïque.

Les isoprénoïdes liés à une cystéine comprennent généralement de 6 à 20 atomes de carbone. Avantageusement, on utilise dans le cadre de l'invention le farnésyl-cystéine, le géranyl-géranyl-cystéine ou le mévalonate-cystéine.

Comme hormone hydrophobe, on utilise avantageusement la progestérone ou le 2-méthoxyoestradiol.

Comme dérivé de la cystéine, on utilise avantageusement l'homocystéine ou la méthionine.

Les indolealkylamines et catécholamines utilisées dans le cadre de l'invention comprennent notamment le tryptophane, le 5-méthoxtryptophane, la sérotonine, la tryptamine, la 5-méthoxytryptamine, la mélatonine, la phénylalanine, la 3,4-dihydroxyphénylalanine et la tyrosine.

Comme acides aminés, on utilise de préférence l'histidine, la glycine, l'aspartate ou l'omithine.

Les acides amino(C₁-C₅)alkylsulfoniques ou sulfiniques utilisés selon l'invention incluent notamment la taurine, l'homotaurine et l'hypotaurine.

Les diamines utilisées de manière préférentielle dans le cadre de l'invention sont la putrescine, la cadavérine, la spermine et la spermidine.

Avantageusement, dans les conjugués monofonctionnels de la polylysine, la molécule utilisée est un (di)acide gras tel que défini précédemment. Dans les conjugués polyfonctionnels de la polylysine, au moins une des molécules utilisées est avantageusement un (di)acide gras, tel que défini précédemment, étant entendu que plusieurs (di)acides gras différents peuvent être utilisés. De préférence, ledit (di)acide gras est choisi parmi l'acide myristique, l'acide palmitique, l'acide stéarique, l'acide oléique ou l'acide azélaïque.

Selon un autre aspect, l'invention concerne également les compléments alimentaires et vitaminés comprenant, à titre de principe actif, au moins un conjugué monofonctionnel et/ou polyfonctionnel de la polylysine défini ci-dessus, notamment au moins un conjugué entre un (di)acide gras et la polylysine.

Les méthodes de couplage entre lesdites molécules et la polylysine, pour l'obtention de ces conjugués sont les méthodes de couplage chimique classiques bien connues de l'homme du métier, entre un groupe fonctionnel de chaque molécule et un groupe fonctionnel de la polylysine. Ces couplages sont réalisés par l'intermédiaire d'un agent de couplage, choisi par exemple parmi le glutaraldéhyde, l'anhydride succinique ou glutarique, le carbodiimide, le chloroformiate d'éthyle,ou l'hexaméthylène diisocyanate. Des exemples de telles méthodes de couplage sont notamment celles décrites dans Geffard et al. (16). On peut également coupler les molécules à la polylysine par simple adsorption. Des exemples de méthodes de couplage appropriées sont décrites en détail dans les préparations ci-après.

A titre illustratif, le couplage entre lesdites molécules et la polylysine peut être effectué entre une fonction amine de la polylysine et une fonction carboxylique desdites molécules.

Ainsi, dans le cas des acides gras, notamment l'acide myristique, l'acide palmitique, etc., ainsi que dans le cas des isoprénoïdes liés à une cystéine, notamment de la farnésylcystéine, la liaison avec la polylysine est effectuée entre une fonction amine de cette dernière et la fonction carboxylique des molécules susmentionnées.

De même, dans le cas de la cystéine et ses dérivés, la liaison avec la polylysine se fait avantageusement entre une fonction amine de cette dernière et la fonction acide de ces molécules.

Alternativement, la cystéine et ses dérivés peuvent être préalablement activés par couplage avec l'anhydride succinique ou glutariquc, la liaison se faisant alors entre une fonction amine de la polylysine et la fonction acide libre de la molécule succinylée ou glutarylée. Ou bien encore, la cystéine et ses dérivés peuvent être liés à la polylysine par réaction avec le glutaraldéhyde, la réaction se faisant notamment comme décrit par Geffard et al. (17).

Dans le cas du cholestérol et de ses dérivés, le couplage avec la polylysine est avantageusement réalisé par l'intermédiaire du groupe hydroxyle du cholestérol.

Avantageusement le cholestérol et ses dérivés sont adsorbés sur la polylysine.

Dans le cas des hormones hydrophobes, le couplage avec la polylysine est avantageusement réalisé par l'hexaméthylène diisocyanate.

Dans le cas de la vitamine A (acide rétinoïque), la liaison entre cette molécule et la polylysine s'effectue entre la fonction amine de cette dernière et la fonction acide de la molécule.

Dans le cas de la vitamine C (acide ascorbique), la liaison entre cette molécule et la polylysine s'effectue entre la fonction amine de cette dernière et la fonction oxo de la molécule.

Dans le cas de la vitamine E (α-tocophérol), la liaison entre cette molécule et la polylysine s'effectue entre la fonction amine de cette dernière et la fonction acide libre du succinate acide de la molécule.

Dans le cas des acides aminoalkylsulfoniques ou sulfiniques, notamment la taurine, l'homotaurine et l'hypotaurine, la liaison entre ces molécules et la polylysine s'effectue par activation préalable des molécules avec un anhydride d'acide (succinique ou glutarique), ou bien par couplage avec le glutaraldéhyde.

Dans le cas des acides aminés, de certains composés indolcalkylamines, notamment le tryptophane, et des catécholamines, on peut effectuer la liaison entre ces molécules et la polylysine soit directement, soit par couplage avec le carbodiimide, le glutaraldéhyde, un anhydride d'acide ou un chlorure d'acide, par exemple le chloroformiate d'éthyle.

Dans le cas des diamines, on peut effectuer la liaison entre ces molécules et la polylysine par couplage avec le glutaraldchyde ou un anhydride d'acide. La liaison entre la camitine ou la carnosine d'une part, et la polylysine d'autre part, s'effectue par couplage avec le carbodiimide.

Les conjugués selon l'invention sont utilisables notamment pour la préparation de compositions ou associations pharmaceutiques utiles dans le traitement de la dégénérescence neuronale, des neuropathies infectieuses, traumatiques et toxiques, des affections dégénératives à caractère autoimmun, des troubles ncurodégénératifs consécutifs à des affections génétiques et des affections proliférativcs et plus particulièrement dans les indications suivantes : troubles de la mémoire, démence vasculaire, troubles postencéphalitiques, troubles post-apoplectiques, syndromes post-traumatiques dus à un traumatisme crânien, troubles dérivant d'anoxies cérébrales, maladie d'Alzheimer, démence sénile, démence subcorticale telle que la chorée de Huntington et la maladie de Parkinson, démence provoquée par le SIDA, neuropathies dérivées de morbidité ou dommage des nerfs sympathiques ou sensoriels, maladies cérébrales telles que l'oedème cérébral, dégénérescences spinocérébelleuses, neuropathies consécutives à une maladie de Lyme, affections présentant des troubles neurodégénératifs telles que la maladie de Charcot-Marie-Tooth, sclérose latérale amyotrophique, sclérose en plaques, épilepsies, migraines, polyarthrites, diabète insulino-dépendant, lupus érythémateux disséminé, thyroïdite de Hashimoto, maladie de Horton, dermatomyosite et polymyosite, ainsi que spondylarthrite ankylosante, infection à VIH et cancers.

L'invention concerne aussi une méthode de traitement de ces maladies à l'aide des conjugués de la polylysine définis précedemment.

Dans le cadre de la présente invention, on entend par association pharmaceutique plusieurs mélanges de conjugués de la polylysine, conditionnés de manière distincte, qui peuvent être administrés simultanément ou séparément par exemple de manière séquentielle. Ces mélanges se présentent dans des conditionnements distincts, identiques ou différents, en combinaison avec un véhicule pharmaceutiquement acceptable. Les compositions pharmaceutiques selon l'invention contiennent, à titre de principe actif, un mélange de conjugués de la polylysine tels que définis précédemment, en combinaison avec un véhicule pharmaceutiquement acceptable.

L'administration des conjugués selon l'invention s'effectue préférablement par voie parentérale. La quantité de principe actif à administrer dans le traitement des troubles ci-dessus selon la méthode de la présente invention dépend bien évidemment de la nature et de la gravité des affections à traiter ainsi que du poids du malade. Néanmoins, les doses unitaires préférées comprendront généralement de 0,1 à 2 ml, de préférence de 0,5 à 1 ml, comme par exemple 0,6 ; 0,8 et 1 ml, de solution de conjugués, à raison de 0,3 à 0,6 mg de conjugués/ml. Ces doses unitaires seront normalement administrées 1 à 4 fois par jour, mais on peut également envisager une administration plus espacée dans le temps, par exemple tous les 2 à 3 jours.

Les conjugués selon l'invention peuvent se présenter sous forme de solutions aqueuses contenant les doses unitaires indiquées ci-dessus dans un véhicule aqueux, isotonique ou stérile qui contient éventuellement des agents de dispersion et/ou des mouillants pharmacologiquement compatibles. Ces solutions sont conditionnées de manière appropriée pour permettre une administration parentérale, par exemple une administration intraveineuse, intramusculaire ou sous-cutanée, ou l'introduction dans un dispositif de perfusion intraveineuse, ou bien encore une administration par un système d'implant permettant la perfusion sous-cutanée.

On peut également administrer les conjugués selon l'invention par voie orale, intranasale, rectale ou bien percutanée. Dans ce cas, les formes unitaires d'administration sont préparées de manière conventionnelle selon les techniques classiques connues de l'homme du métier, avec les excipients couramment utilisés dans ce domaine.

A titre d'exemples de composition pharmaceutique selon l'invention, on peut mentionner : (les abréviations utilisées sont explicitées ci-après).
* une composition qui comprend les conjugués suivants : L-DOPA-G-PL, αtoco-PL, Ole-PL-Farcys, PO-PL-Ole, Myr-PL-Ole, Cys-AG-PL ou Cys-AS-PL, Homocys-AG-PL ou Homocys-AS-PL, Met-AG-PL ou Mct-AS-PL, Met-G-PL, Cys-G-PL, Homocys-G-PL, Laur-PL-Ole, GABA-AG-PL, 5MT-AG-PL, His-PL, Cys-PL-Nac, Homocys-PL-Nac, Mct-PL-Nac, utile notamment pour le traitement de la maladie de Parkinson.
* une composition qui comprend les conjugués suivants : GABA-G-PL, PO-PL-Aze, Pal-PL-Farcys, Myr-PL-Farcys, W-AG-PL, Ole-PL-Pal, Myr-PL-Ole, αtoco-PL, Aze-PL-Lin2, utile notamment pour le traitement des épilepsies.
* une composition qui comprend les conjugués suivants : HW-AG-PL, W-AG-PL, MT-AG-PL, αtoco-PL, Cys-AG-PL, Met-AG-PL, Homocys-AG-PL, utile notamment pour le traitement des migraines essentielles.

A titre d'exemples d'association pharmaceutique selon l'invention, on peut mentionner:
* une association qui comprend dans deux conditionnements distincts, d'une part les conjugués suivants (1a) : Farcys-PL-Pal, Pal-PL-Myr, Farcys-PL-Myr, Lin2-PL-Ole, Met-PL-Nac, Cys-PL-Nac, Homocys-PL-Nac, Farcys-PL-Ole, Met-AG-PL, Pal-PL-Ole, Myr-PL-Ole, Tau-AG-PL, VitC-PL, Tau-G-PL, Stca-PL-Farcys, ACh-AG-PL, α toco-PL, Aze-PL-Ole ; et d'autre part les conjugués suivants (1b): Farcys-PL-Pal, Farcys-PL-Myr, Pal-PL-Myr, Aze-PL-Ole, Pal-PL-Ole, Farcys-PL-Ole, PO-PL-Aze, Ole-PL-Myr, Stea-PL-Farcys, Ole-PL-Laur, utile notamment pour le traitement des neuropathies post-Lyme et de la spondylarthrite ankylosante.
* une association qui comprend dans deux conditionnements distincts, d'une part les conjugués suivants (2a) : Myr-PL-Pal, Pal-PL-Ole, Myr-PL-Ole, Tau-AS-PL ou Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL ou Met-AG-PL, Homocys-AS-PL ou Homocys-AG-PL, Cys-G-PL, Homocys-G-PL, Met-G-PL, CHO-PL, 5MT-AG-PL, W-AG-PL, ACh-AG-PL ; et d'autre part les conjugués suivants (2b) : Pal-PL-Ole, Tau-AS-PL ou Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL ou Met-AG-PL, Homocys-AS-PL ou Homocys-AG-PL, Cys-AG-PL, Cys-G-PL, Met-G-PL, Homocys-G-PL, Met-G-PL, CHO-PL, W-AG-PL, ACh-AG-PL, utile notamment pour le traitement da la sclérose latérale amyotrophique.
* une association qui comprend dans trois conditionnements distincts :
   3a) les conjugués suivants : Myr-PL-Pal, Pal-PL-Ole, Myr-PL-Ole, Tau-AS-PL ou Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL ou Met-AG-PL, Homocys-AS-PL ou Homocys-AG-PL, Cys-G-PL, Homocys-G-PL, Met-G-PL, CHO-PL, 5MT-AG-PL, W-AG-PL, ACh-AG-PL ;
   3b) les conjugués suivants : Cys-AG-PL, Met-AG-PL, Homocys-G-PL, Cys-PL-Nac, Homocys-PL-Nac, Met-PL-Nac, Tau-AG-PL, Tau-G-PL, Met-G-PL, αtoco-PL, αtoco-PL-Ret ;
   et 3c) les conjugués suivants : Farcys-PL-Ole, Aze-PL-Ole, Aze-PL-PO, Met-PL-Nac, Cys-PL-Nac, Homocys-PL-Nac, αtoco-PL, Lin2-PL-Ole, Ret-PL, Ole-PL-Laur, αtoco-PL-Ret, Tau-G-PL, ACh-AG-PL, PRO-PL, GABA-G-PL,
      utile notamment pour le traitement de la choréc de Huntington.
* une association qui comprend dans quatre conditionnements distincts :
   4a) les conjugués suivants : Farcys-PL-Ole, PO-PL-Ole, Aze-PL-Ole, Aze-PL-PO, Met-PL-Nac, Homocys-PL-Nac, αtoco-PL, VitC-PL, Tau-AG-PL, Lin2-PL-Ole, CHO-PL, Ole-PL-Laur, 5MT-AG-PL, Tau-G-PL, ACh-AG-PL ;
   4b) les conjugués suivants : Farcys-PL-Pal, Farcys-PL-Myr, Pal-PL-Myr, Aze-PL-Ole, Pal-PL-Ole, Farcys-PL-Ole, PO-PL-Azc, Olc-PL-Myr, Stea-PL-Farcys, Ole-PL-Laur ;
   4c) les conjugués suivants : Farcys-PL-Olc, Aze-PL-Olc, Azc-PL-PO, Met-PL-Nac, Cys-PL-Nac, Homocys-PL-Nac, αtoco-PL, Lin2-PL-Ole, Ret-PL, Ole-PL-Laur, αtoco-PL-Ret, Tau-G-PL, ACh-AG-PL, PRO-PL, GABA-G-PL ;
   et 4d) les conjugués suivants : Farcys-PL-Olc, Aze-PL-Ole, Aze-PL-PO, αtoco-PL, VitC-PL, Tau-AG-PL, Lin2-PL-Ole, CHO-PL, Laur-PL-Ole,
      utile notamment pour le traitement de la sclérose en plaques.
* une association qui comprend dans trois conditionnements distincts :
   5a) les conjugués suivants : Farcys-PL-Pal, Pal-PL-Myr, Farcys-PL-Myr, Lin2-PL-Ole, Met-PL-Nac, Cys-PL-Nac, Homocys-PL-Nac, Farcys-PL-Ole, Met-AG-PL, Pal-PL-Ole, Myr-PL-Ole, Tau-AG-PL, VitC-PL, Tau-G-PL, Stea-PL-Farcys, ACh-AG-PL, αtoco-PL, Aze-PL-Ole ;
   5b) les conjugués suivants : Farcys-PL-Pal, Farcys-PL-Myr, Pal-PL-Myr, Aze-PL-Ole, Pal-PL-Ole, Farcys-PL-Ole, PO-PL-Aze, Ole-PL-Myr, Stea-PL-Farcys, Ole-PL-Laur ;
   et 5c) les conjugués suivants : Farcys-PL-Pal, Pal-PL-Myr, Farcys-PL-Myr, Farcys-PL-Ole, Myr-PL-Ole, Stea-PL-Farcys, ACh-AG-PL, αtoco-PL,
      utile notamment pour le traitement des polyarthrites.
* une association qui comprend dans deux conditionnements distincts, d'une part les conjugués suivants (6a) : Lin2-PL-Farcys, Stea-PL-Farcys, Myr-PL-Farcys, Pal-PL-Farcys, Ole-PL-Farcys, Cys-AG-PL, Met-AG-PL, Homocys-AG-PL, CHO-PL, Homocys-PL-Nac, Cys-PL-Nac, αtoco-PL-Ret, αtoco-PL, VitC-PL, Ole-PL-Laur, Met-G-PL, Met-PL-Nac, Homocys-PL-Nac, Cys-G-PL; et d'autre part les conjugués mentionnés au point 3c) ci-dessus, utile notamment pour le traitement de l'infection à VIH.
* une association qui comprend dans trois conditionnements distincts :
   7a) les conjugués suivants : PRO-PL, Ret-PL, αtoco-PL, αtoco-PL-Ret, W-AG-PL, 5MT-AG-PL, Hist-PL;
   7b) les conjugués mentionnés au point 1b) ci-dessus; et
   7c) les conjugués suivants : L-DOPA-G-PL, L-DOPA-AG-PL, 5MT-AG-PL, HW-AG-PL, Tyr-AG-PL,
utile notamment pour le traitement des cancers.

Selon un autre aspect, l'invention concerne également les conjugués monofonctionnels et polyfonctionnels entre d'une part la polylysine et d'autre part une (plusieurs) molécule(s) choisie(s) parmi :
- les (di)acides gras à chaîne linéaire ou ramifiée, saturés ou insaturés, comprenant généralement de 4 à 24 atomes de carbone ;
- les composés impliqués dans le mécanisme d'ancrage des protéines aux membranes cellulaires, ces composés intervenant notamment dans le cycle du mévalonate, en particulier les isoprénoïdes liés à une cystéine ;
- le cholestérol et ses dérivés, notamment les hormones hydrophobes;
- la vitamine A, la vitamine C, la vitamine E ou un de leurs dérivés ;
- la cystéine et ses dérivés, de formule :

   R₁S-R₂-CH(NH₂)-COOH
dans laquelle R₁ représente H ou CH₃ et R₂ représente un C₁-C₃ alkylène.
- les indolealkylamines;
- les catécholamines;
- les acides aminés de formule :

   R₃-CH(NH₂)-COOH
dans laquelle R₃ représente l'hydrogène, un groupe imidazol-5-ylméthyle, un groupe carboxyméthyle ou un groupe aminopropyle ;
- les acides amino(C₁-C₅)alkylsulfoniqucs ou sulfiniques;
- la carnitine ou la carnosine;
- les diamines de formule :

   ₂HN-A-NH₂
dans laquelle A représente un (C₁-C₆)alkylène ou un groupe -(CH₂)ₘ-NH-B-(CH₂)ₚ- dans lequel m et p, indépendamment l'un de l'autre, sont des entiers allant de 1 à 5, et B représente rien ou un groupe -(CH₂)ₙ-NH-, n étant un entier allant de 1 à 5 ;
- l'acétylcholine et
- l'acide γ-aminobutyrique.
à la condition que lorsque le conjugué est monofonctionnel, la molécule est choisie parmi :
- une hormone hydrophobe ;
- la vitamine A, la vitamine C, la vitamine E ou un de leurs dérivés ;
- un acide aminé de formule :

   R₃-CH(NH₂)-COOH
dans laquelle R₃ représente l'hydrogène, un groupe imidazol-5-ylméthyle ou un groupe aminopropyle ;
- un acide amino(C₁-C₅)alkylsulfonique ou sulfinique;
- la carnitine ou la carnosine; ou
- une diamine de formule:

   ₂HN-A-NH₂
dans laquelle A représente un (C₁-C₆)alkylène ou un groupe -(CH₂)ₘ-NH-B-(CH₂)ₚ- dans lequel m et p, indépendamment l'un de l'autre, sont des entiers allant de 1 à 5, et B représente rien ou un groupe -(CH₂)ₙ-NH-, n étant un entier allant de 1 à 5 ;

Les préparations et exemples qui suivent illustrent l'invention sans toutefois en limiter la portée.

Les préparations illustrent la synthèse des conjugués, qui sont identifiés par l'agent de couplage utilisé, et les exemples sont relatifs aux compositions et associations pharmaceutiques de l'invention. Dans ceux-ci, les abréviations suivantes ont été utilisées :
SMT = 5-méthoxytryptamine ;
ACh = acétylcholine ;
AG = anhydride glutarique ;
AS = anhydride succinique ;
G = glutaraldehyde réduit :
αtoco = α-tocophérol ;
Aze = acide azélaïque ;
CHO = cholestérol ;
Cys = cystéine ;
DMF = diméthylformamide ;
DMSO = diméthylsulfoxyde ;
ECF = chloroformiate d'éthyle
Farcys = farnésyl cystéine ;
GABA = acide γ-aminobutyrique ;
His = histamine ;
Hist = histidine ;
Homocys = homocystéine ;
HW = 5-hydroxytryptophane ;
Laur = acide laurique ;
L-DOPA = L-3,4-dihydroxyphénylalanine ;
Lin 2= acide α-linoléique ;
MBS = métabisulfite de sodium
MES = acide morpholinoéthanol sulfonique
Met = méthionine ;
Myr = acide myristique ;
Pal = acide palmitique ;
Nac = N-acétylé ;
Ret = acide rétinoïque ;
Ole = acide oléique ;
PO = acide palmitoléique ;
Stea= acide stéarique ;
Tau = taurine ;
TEA = triéthylamine ;
VitC = acide ascorbique ;
W = tryptophane ;
PRO = progestérone ;
2MO = 2-méthoxyoestradiol;
AI = anticorps monoclonal anti-idiotypique
PL = polylysine.

### Préparations

### I. Conjugués glutaraldéhyde:

Cette préparation illustre notamment la préparation des conjugués de Cystéine ou de ses dérivés avec la polylysine selon le schéma réactionnel ci-après :

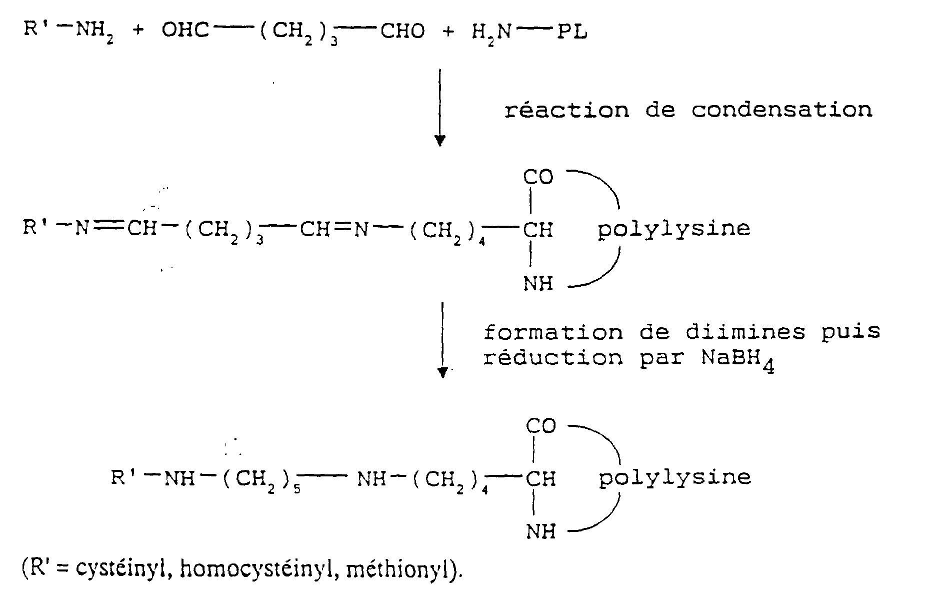

20 mg d'haptène sont repris dans 1 ml de tampon acétate 1,5 M (pH8) avec 1 *µ*l de traceur radioactif (³H tyrosine : ref. NET 127, Dupont de Nemours). Dans le cas de conjugués catécholaminergiques, il faut rajouter quelques grains de MBS. Dans le cas du tryptophane, il faut solubiliser ce dernier dans 1 ml de DMSO (Prolabo) et 1 ml d'acétate de sodium (Prolabo).

Séparément, 40 mg de polylysine sont repris dans 2 ml de tampon acétate 1,5 M.

A la solution d'haptène, on ajoute 1 ml d'une solution de glutaraldéhyde (Prolabo) à 5 %, et environ 10 secondes après, la polylysine. La réaction de couplage se traduit par une coloration jaune ; dès que celle-ci est atteinte, la réaction est stoppée par addition de quelques gouttes d'une solution de NaBH₄ (Aldrich) (quelques grains dans de l'eau, de manière à avoir une solution qui "bulle").

Dans le cas particulier de la taurine, il faut rajouter 1 ml de glutaraldéhyde à 1 % seulement.

Les conjugués sont purifiés par dialyse contre de l'eau, sauf pour les catécholamines, où il faut rajouter du MBS à la solution de dialyse.

Les conjugués ainsi obtenus sont désignés par : R-G-PL (R = haptène, G = résidu glutaraldéhyde réduit).

### II. Conjugués avec un bras succinyl ou glutaryl

C'est le cas par exemple de l'α-tocophérol (vitamine E), de la cystéine et ses dérivés, ou bien encore de la 5-méthoxytryptamine, de la taurine, de l'homotaurine :
* Cys-AS ou AG-PL 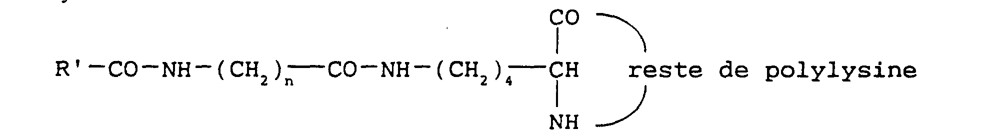 R' = cystéinyl, homocystéinyl, méthionyl, avec n = 2 dans le cas de l'acide ou anhydride succinique, n = 3 dans le cas de l'acide ou anhydride glutarique.
* αtoco-AS-PL 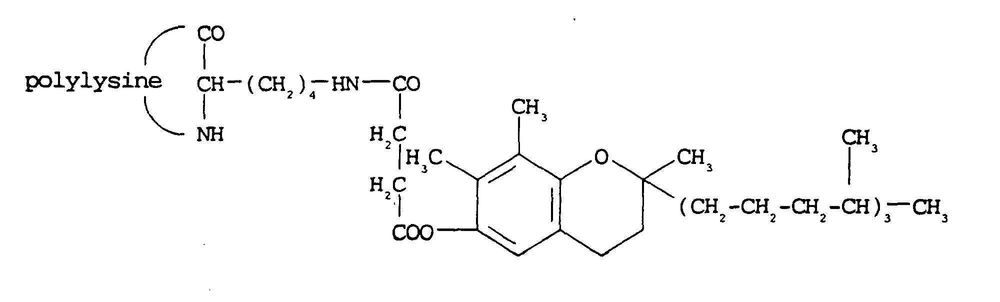
* 5MT-AS ou AG-PL 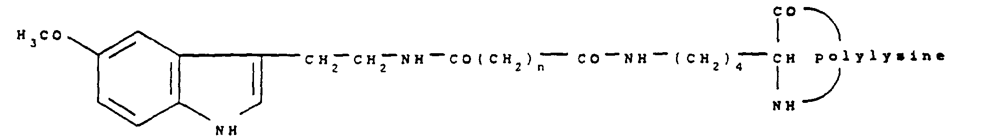 n = 2 ou 3
* (homo)tau-AS ou AG-PL 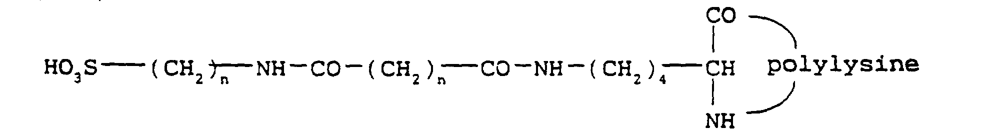 n = 2, 3

40 mg d'haptène sont solubilisés dans 1 ml d'eau avec 1 *µ*l de traceur radioactif (³H tyrosine). Dans le cas de la 5-méthoxytryptamine, sa solubilisation n'est possible qu'en mettant 200 *µ*l de DMSO. On ajoute 34 mg d'anhydride glutarique ou succinique (Sigma) et aussitôt 340 *µ* l de NaOH 1M (Prolabo) de manière à être à pH 7. Les conjugués sont ensuite lyophilisés.

L'accrochage de ces conjugués sur la polylysine se fait par le chloroformiate d'éthyle (Fluka) à 4° C. Les haptènes -glutaryl ou -succinyl sont repris dans 1,6 ml de DMF anhydre (Prolabo) et 20 *µ*l de TEA anhydre (Merck) (pas de solubilisation complète). L'activation des COOH se fait par 400 *µ*l d'ECF dilué au 1/16 dans du DMF anhydre pendant 5 minutes. Puis, on ajoute une solution de PL (80 mg dissous dans 2 ml d'eau et 20 *µ*l de TEA anhydre).

La purification des conjugués est faite par dialyse contre de l'eau.

Les conjugués ainsi obtenus sont désignés par : R-AS ou AG-PL (R = haptène, AS = résidu succinyl, AG = résidu glutaryl).

### III. Conjugués carbodiimide

50 mg d'haptène et 50 mg de polylysine sont solubilisés dans 1 ml de tampon MES 0,2 M pH 5,4.

On ajoute petit à petit, à cette solution, 200 mg de carbodiimide (Sigma) en environ 10 minutes. Il faut vérifier à chaque addition le pH, et l'ajuster à 5,4.

La purification des conjugués est faite par dialyse contre de l'eau.

Les conjugués ainsi obtenus sont désignés par : R-PL (R = haptène lié par -CO-NH-).

### IV. Conjugués chloroformiate d'éthyle (molécules hydrophiles)

* VitC-PL: 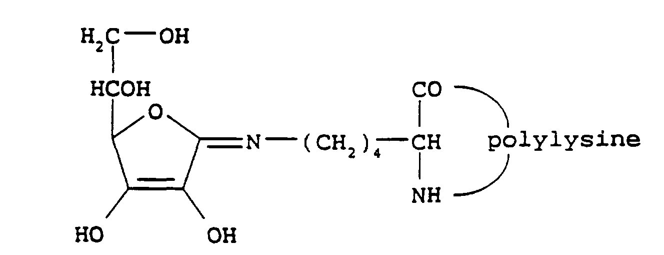 40 mg d'haptène sont repris dans 1,6 ml de DMF anhydre et 20 *µ*l de TEA anhydre. L'activation des COOH se fait par 400 *µ*l d'ECF dilué au 1/16 dans du DMF anhydre pendant 5 minutes. Au bout de ce temps, on ajoute une solution de PL (80 mg dissouts dans 2 ml d'eau et 20 *µ*l de TEA anhydre).
* Cystéine, homocystéine, méthionine :
   40 mg d'haptène sont repris dans 2 ml de méthanol anhydre et 20 *µ*l de TEA anhydre (solubilisation difficile). L'activation des COOH se fait avec 1 ml d'ECF dilué au 1/16 dans du DMF anhydre pendant 5 minutes. Au bout de 5 minutes, on ajoute une solution de PL (80 mg dissous dans 2 ml d'eau et 20 *µ*l de TEA anhydre).
* carnitine :
   On s'assure de la bonne déshydratation de la carnitine en la lyophilisant. Le couplage doit se faire ensuite très rapidement, car la carnitine est très hydroscopique. Le protocole est le même que pour la vitamine C.
   La purification des conjugués est faite par dialyse contre de l'eau.
   Les conjugués ainsi obtenus sont désignés par : R-PL (R = haptène).

### V. Conjugués chloroformiate d'éthyle (acides gras, farnesyl cystéine, acide rétinoïque et α-tocophérol)

### * Cas de la farnesyl cystéine :

La farnésyl cystéine est le résultat de la condensation du famésol : 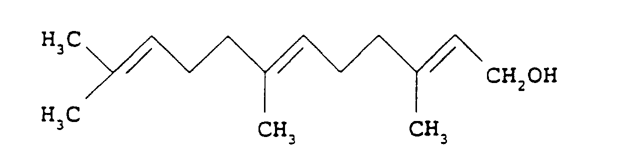 avec la cystéine : 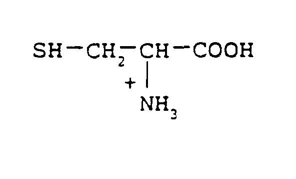 par liaison thioéther. 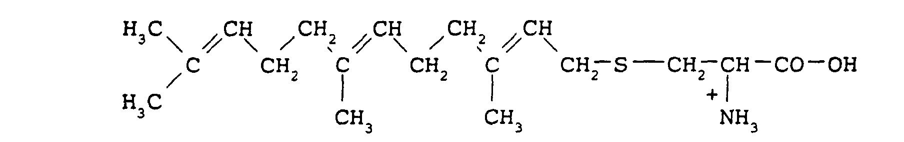 Cette molécule est ensuite liée à la polylysine : 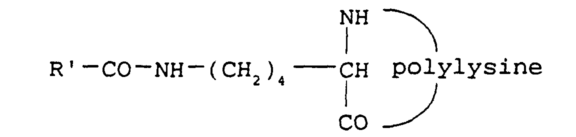 (R' = résidu de farnésylcystéine)

On utilise 20 mg d'haptènc pour les acidcs gras (40 mg pour l'acide azélaique), 40 mg pour la farnésylcystéine, 40 mg pour l'acide rétinoique ou 60 mg pour l'α-tocophérol. Les haptènes sont solubilisés dans 1 ml de méthanol anhydre et 20 *µ*l de TEA anhydre avec 1 *µ*l de marqueur radioactif (acide palmitique ¹⁴C : réf. C14 NECQ, Dupont de Nemours). Puis on laisse le mélange sous atmosphère sèche pendant 30 minutes.

On active ensuite ce mélange par 200 *µ*l de chloroformiate d'éthyle dilués au 1/16 dans du DMF anhydre , pendant 15 minutes à 4° C. Puis on rajoute la solution de PL (80 mg dans 4 ml de tampon phosphate 10⁻²M contenant CaCl₂ 10⁻³M et 40 *µ*l de TEA anhydre).

La réaction terminée, le conjugué est purifié par dialyse. On transfère les solutions obtenues dans des tubes de dialyse (Tube dialyse Visking, Prolabo), dont les pores laissent passer les molécules non couplées (PM<12 à 14 000 Da). Pour éliminer le maximum de molécules non couplées, la 1ère solution de dialyse comprend : 1/3 de DMF, 1/3 de méthanol, 1/3 de tampon phosphate 10⁻²M avec du CaCl₂ à 10⁻³M. Deux autres dialyses sont faites, avec une solution de tampon phosphate 10⁻²M contenant du CaCl₂ 10⁻³M, pendant 24h. Le rôle du CaCl₂ est de rendre les conjugués plus solubles par interaction des ions Ca²⁺ avec les chaînes aliphatiques. Mais ce tampon peut être remplacé par un tampon guanidine 10 mM pH7.

Les conjugués ainsi obtenus sont désignés par : R-PL (R = haptène).

### VI. Acétylation des conjugués farnésylcystéine ou thiols couplés par la carbodiimide

Le pH des conjugués est ramené à 10 avec de la soude 1M. Puis on ajoute un mélange de 4 *µ*l d'anhydride acétique (Prolabo) et de 500 *µ*l de DMSO anhydre. L'addition de 40 *µ*l de NaOH 1M permet de neutraliser la réaction.

Les conjugués sont ensuite dialysés contre de l'eau.

Les conjugués ainsi obtenus sont désignés par : R-PL-Nac (R = haptène, Nac = N-acétylé).

### VII. Conjugués cholestérol ou acide rétinoïque par adsorption

### * Cas de l'acide rétinoïque :

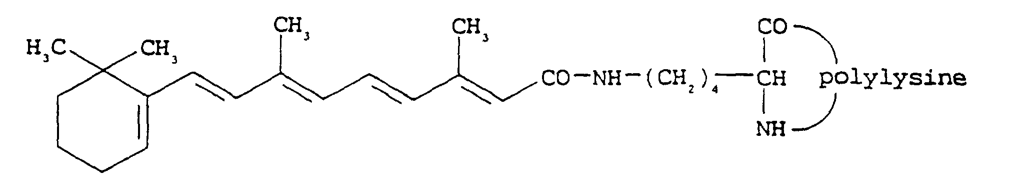

40 mg d'haptène sont solubilisés avcc 2 ml de méthanol anhydre et 2 ml de DMF anhydre avec 1 *µ*l de traceur radioactif (cholesterol : réf. NET 725, Dupont de Nemours). Puis on ajoute goutte à goutte 80 mg de FL dissous dans 2 ml de tampon acétate 1,5 M. On agite bien puis on purifie les conjugués par dialyse. La première solution de dialyse est constituée d'un mélange 1/3 méthanol, 1/3 DMF et 1/3 eau. Les deux solutions suivantes comprennent uniquement de l'eau.

Les conjugués ainsi obtenus sont désignés par : R-PL (R = haptène).

### VIII. Conjugués hexaméthylène diisocyanate

5 mg de 2-méthoxyocstradiol sont repris dans 1 ml de DMSO avec 1 *µ*l d'hormone radioactive (progestérone : réf. N381, Dupont de Nemours). On ajoute 10 mg de PL dissous dans 1 ml de tampon phosphate 0,2 M pH10, et 50 *µ*l d'hexaméthylène diisocyanate (Fluka). On ajuste le pH à 10 avec de la soude 1N.

Pour la progestérone, le protocole est le même, mais on part de 40 mg de progestérone, 80 mg de PL et 200 *µ*l d'hexaméthylène diisocyanate.

La purification des conjugués se fait par dialyse, la 1ère solution est constituée d'un mélange DMF/DMSO/eau, les deux suivantes d'eau.

Les conjugués ainsi obtenus sont désignés par : R-PL (R = haptène).

De manière générale, le petit matériel dans lequel se font les réactions de couplage et les purifications des conjugués hydrophobes, est délipidé par rinçage au méthanol et séchage pour éviter toute contamination lipidique extérieure. De plus, dans la synthèse des conjugués ayant un haptène possédant une fonction thiol (cystéine, homocystéine, méthionine, etc..), on ajoute après la dialyse quelques grains de l'haptène correspondant non couplé.

Dans les exemples qui suivent, les conjugués de la polylysine ont été synthétisés suivant le mode opératoire décrit précédemment, en utilisant de la polylysine commercialisée par la société Sigma sous la référence P 7890. A titre d'indication, les rapports de couplage des conjugués utilisés dans les exemples sont rapportés dans le Tableau suivant:

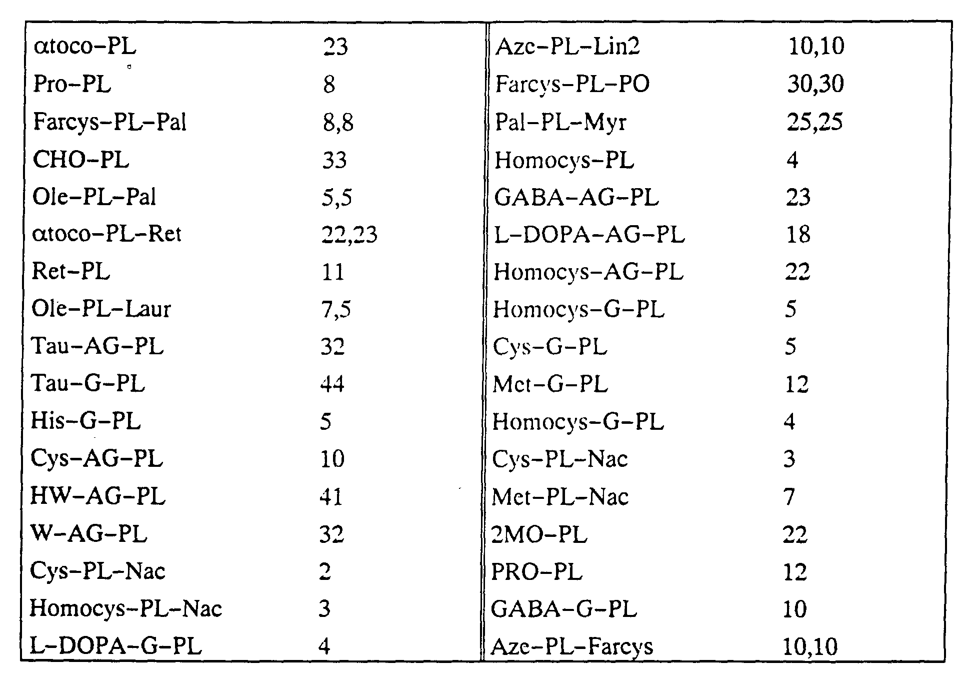

Par rapport de couplage, on entend le nombre de molécules d'haptène(s) conjuguées par molécule de polylysine. Dans le cas des conjugués bifonctionnels, on indique, pour chaque haptène, le nombre respectif de molécules conjuguées.

Exemple 1 : Préparation d'une association pharmaceutique pour le traitement des troubles neurologiques consécutifs à une maladie de Lyme

De nombreuses affections neurologiques sont la conséquence d'infections bactériennes et virales connues ou non. *Borrelia burgdorflei*, est l'agent étiologique de la maladie de Lyme. Il provoque dans un certain nombre de cas des neuropathies.

On a préparé les solutions suivantes:

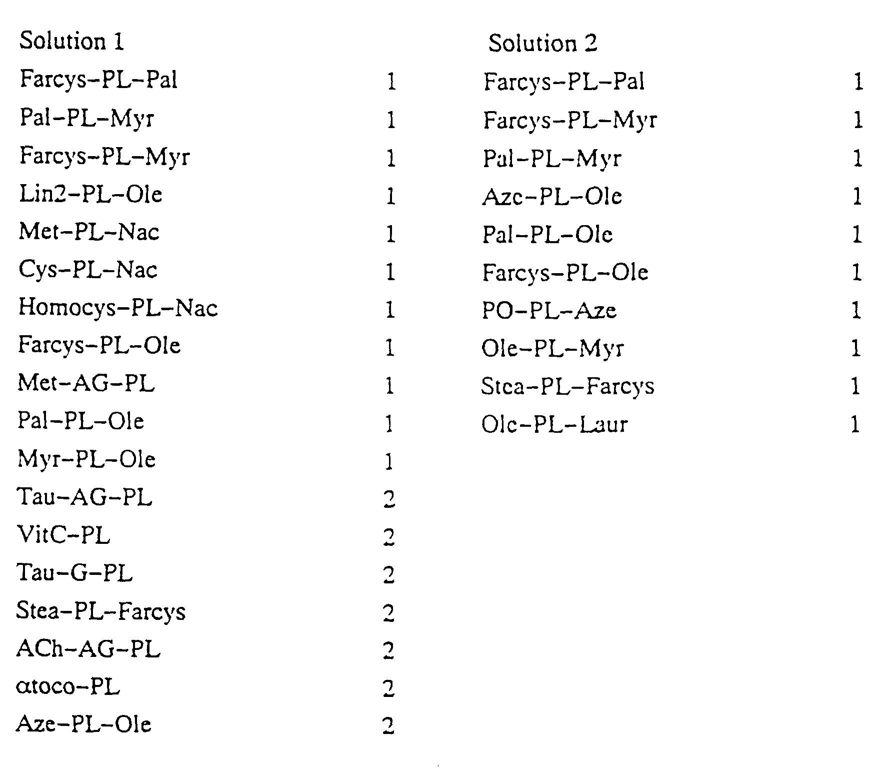

Le chiffre à droite du conjugué monofonctionnel ou bifonctionnel de la polylysine indique la concentration en haptène(s) dudit conjugué dans la solution, étant entendu que la valeur 1 correspond à une concentration égale à 10⁻⁵ M. Ainsi, pour le conjugué bifonctionnel Farcys-PL-Pal, on a:
[Farcys] = 10⁻⁵M
[Pal] = 10⁻⁵M.

De cette manière, on peut déterminer la concentration finale de chaque haptène dans les solutions 1 et 2. Par exemple, la concentration finale en Farcys dans la solution 1 est de 5.10⁻⁵M.

### Procédé de préparation :

On mélange les différentes solutions de conjugués monofonctionnels et bifonctionnels, préparées extemporanément dans du sérum physiologique, de manière à obtenir, après mélange au vortex et dilution dans les solutions finales respectives 1 et 2, les concentrations en haptènes indiquées précédemment. Puis, les solutions sont filtrées séparément sur des filtres Sartorius stériles ayant des pores de 0,22 *µ*m puis réparties dans des flacons stériles distincts ou préparées en solutions buvables distinctes (mélange sirop, huile végétale pure 1ére pression).

Les solutions 1 et 2 sont administrées alternativement par injection intramusculaire ou sous-cutanée lente, à raison de 1 ml de chaque solution 1 à 2 fois par jour, par exemple 2 fois la solution 2 pour 1 fois la solution 1 chaque jour.

On constate chez tous les malades (n = 4) traités selon le protocole décrit ci-dessus, une régression de la neuropathie causée par la maladie de Lyme.

Exemple 2 : Préparation d'une association pharmaceutique pour le traitement de la sclérose latérale amyotrophique

La sclérose latérale amyotrophique (SLA), encore appelée maladie de Charcot, est une affection progressive qui fait partie de la triade des affections dégénératives du système nerveux âgé, les deux autres étant les maladies d'Alzheimer et de Parkinson. Toutes les trois sont caractérisées par une vulnérabilité sélective d'un groupe de neurones disparaissant pour des raisons non encore connues. En effet, les lésions ne sont causées ni par des problèmes vasculaires, ni par des réactions inflammatoires. Dans la SLA, les cellules atteintes sont les motoneurones de la corne antérieure de la moëlle épinière et des noyaux moteurs crâniens : V, VII, IX, XI, XII ; et, les voies motrices (corticospinales et corticobulbaires) qui, dans leur ensemble peuvent être touchées, entraînant secondairement une amyotrophie. L'évolution se fait vers une aggravation progressive, avec installation d'un état grabataire, d'une insuffisance respiratoire par atteinte de la musculature intercostale et diaphragmatique menant rapidement au décès (24 à 26 mois en moyenne dans la majorité des cas). La SLA survient chez l'adulte, homme et femme, entre 40 et 60 ans (on constate 2000 nouveaux cas par an en France). L'incidence de la SLA est égale à celle de la sclérose en plaques (SEP) et est cinq fois supérieure à celle de la maladie de Huntington.

L'étiologie de cette affection demeure encore inconnue. Le modèle qui prévaut aujourd'hui est multifactoricl. Elle résulterait de l'action combinée d'une susceptibilité génétique (5 à 10 % des cas de SLA familiale seraient rattachés à une transmission autosomale localisée sur le chromosome 21), d'un vieillissement accéléré et de facteurs neurotoxiques exogènes et/ou endogènes aboutissant à la destruction sélective des motoneurones.

On a préparé les solutions suivantes:

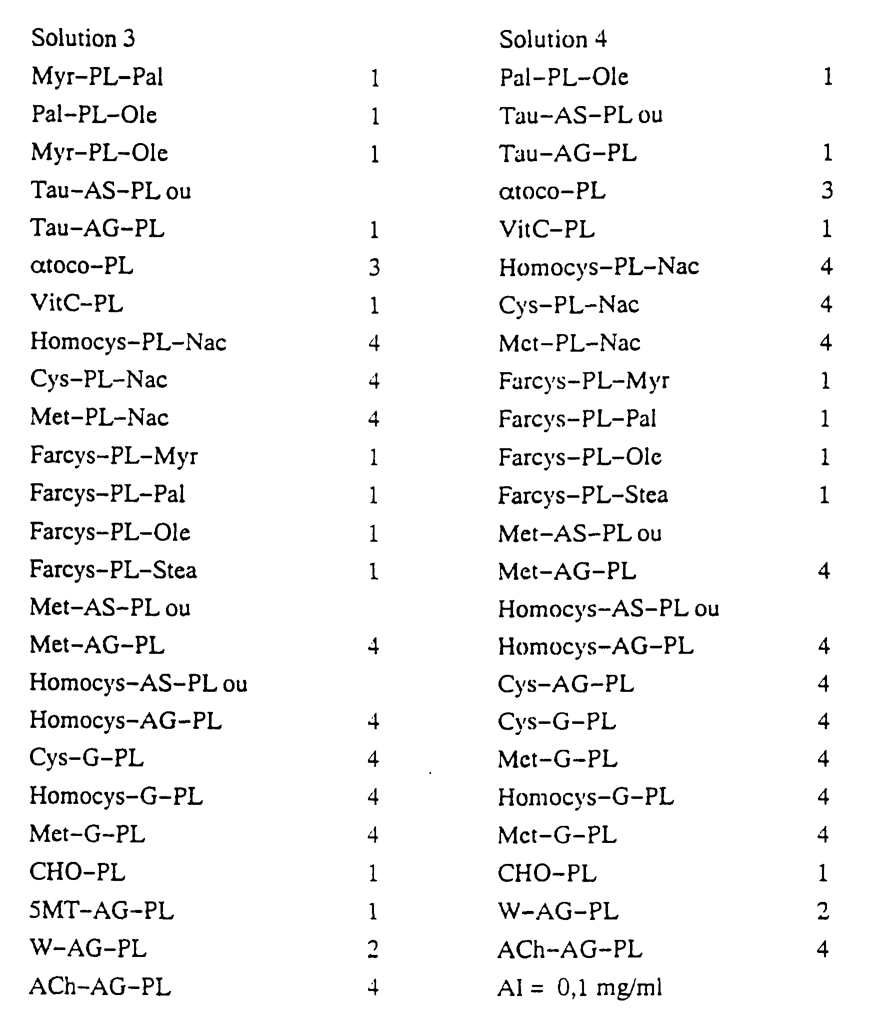

### Procédé de préparation : comme pour l'exemple 1.

Les solutions 3 et 4 sont administrées par injection sous-cutanée lente, à raison de 0,8 ml de chaque solution 2 fois par jour, ou bien 0,8 ml de solution par jour en alternant les solutions 3 et 4 un jour sur deux.

On observe dans 80 % des cas (n = 30) une stabilisation de la symptomatologie ; dans 40 à 50 % des cas une amélioration de la symptomatologie est même constatée (régression des atteintes).

Exemple 3 : Préparation d'une composition pharmaceutique pour le traitement de la maladie de Parkinson

C'est une affection dégénérative du système nerveux central dans laquelle les neurones dopaminergiques de la voie nigro striée sont sélectivement détruits.

La maladie de Parkinson est l'aboutissement d'un processus chronique actif débutant 20 à 30 ans avant l'expression de la maladie, dont l'origine selon les hypothèses récentes est d'ordre multifactoriel. Elle est le résultat de l'action combinée d'une susceptibilité génétique, du vieillissement, et de facteurs neurotoxiques exogènes et endogènes aboutissant à la destruction des systèmes monoaminergiques ascendants. L'analyse post-mortem de sujets atteints de la forme idiopathique ou post-encéphalitique, met en évidence les phénomènes essentiels suivants :
(i) une dégénérescence du faisceau nigrostrié se traduit par l'effondrement des taux de dopamine (DA) et de ses métabolites dans le néostriatum et par une perte neuronale prédominante dans la pars compacta de la substance noire ;
(ii) une déficience en DA dans les neurones de l'aire tegmento-ventrale (ATV) qui sont à l'origine de la voie mésocorticolimbique et qui projettent sur le noyau accumbens. Une diminution moins importante du nombre de neurones DA et des corps de Lewy est observée dans la partie ventrale de l'ATV. La déplétion en DA doit atteindre un certain seuil critique (60 % à 80 %) avant que les symptômes parkinsoniens n'apparaissent. L'akinésie étant le symptôme principal relié au degré de déplétion en DA ;
(iii) la disparition en neurones nigriques s'accompagne de la libération du pigment mélanique et surtout de l'apparition de corps de Lewy, leur présence est un indicateur habituel de cette atteinte neuronale spécifique.

Au niveau évolutif et individuel, la maladie de Parkinson résulterait de l'intervention de ces différents facteurs, tôt dans la vie, chez des sujets possédant une susceptibilité génétique s'exprimant par exemple dans leur capacité anti-oxydative (glutathion réductase, phénotype d'hydroxylation...) conduisant à la réduction du pool neuronal nigrique. La perte neuronale physiologique liée à l'âge aboutirait ensuite à l'expression clinique de la maladie lorsque 80 % des neurones dopaminergiques nigriques manquent.

On a préparé la solution suivante :

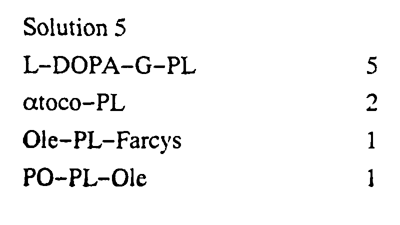

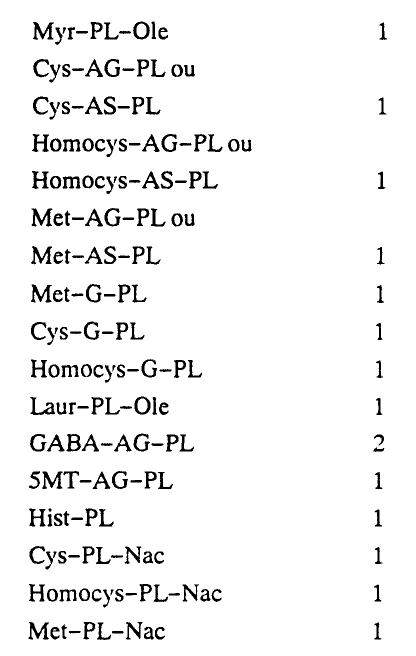

### Préparation : comme pour l'exemple 1.

La solution 5 est administrée par injection sous-cutanée, à raison de 0,4 à 0,8 ml de solution tous les 2 à 3 jours.

On constate une amélioration de la rigidité, de l'akinésie et des tremblements dans 80 % des cas (n = 15), avec une diminution de la prise d'autres traitements.

### Exemple 4 : Préparation d'une association pharmaceutique pour le traitement de la chorée de Huntington

On a préparé les solutions suivantes :

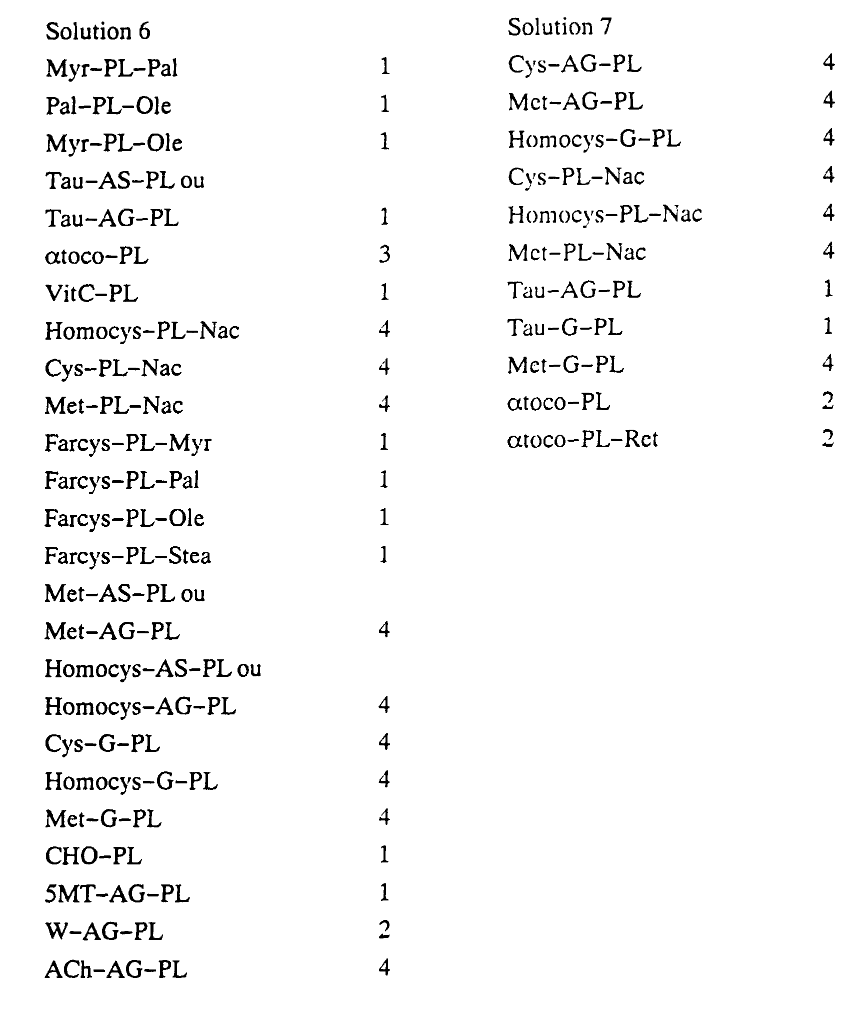

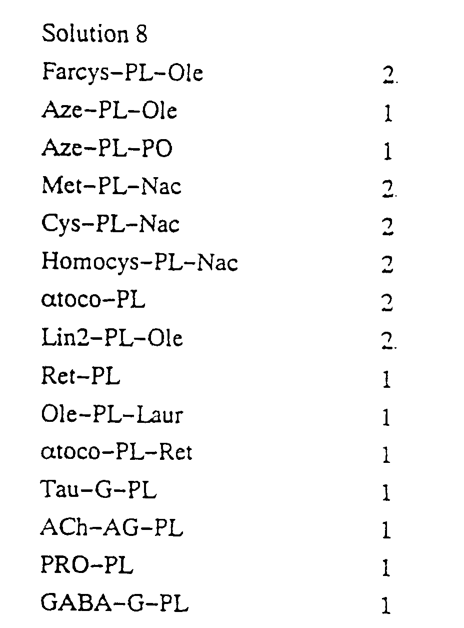

### Préparation: comme pour l'exemple 1.

Les solutions 6, 7 et 8 sont administrées par injection sous-cutanée:
- Soit à raison de 0,6 ml de chaque solution tous les jours ;
- soit alternativement à raison de 0,6 ml d'une solution donnée tous les deux à trois jours (par exemple, on peut administrer la solution 6 le premier jour, la solution 7 le deuxième jour et la solution 8 le troisième jour, etc... ou bien encore on peut administrer la solution 6 le premier jour, la solution 7 le troisième jour et la solution 8 le cinquième jour, etc...).

Sur cinq patients en cours de traitement, on constate une nette amélioration au niveau de la récupération des fonctions mnésiques et de la vivacité relationnelle.

### Exemple 5 : Préparation d'une association pharmaceutique pour le traitement de la sclérose en plaques

En France, plus de 50 000 personnes sont atteintes de sclérose en plaques (SEP), et au moins 2000 nouveaux cas sont enregistrés chaque année. Cette affection débute chez le sujet jeune, habituellement entre 20 et 40 ans. Son incidence est 2 à 3 fois plus forte chez les femmes que chez les hommes. Les symptômes sont variables. Ils peuvent se présenter sous forme : de troubles visuels, d'une faiblesse musculaire, d'une perte de coordination des mouvements ou de contractures... Ils reflètent tous une altération de la conduction nerveuse centrale. Ils peuvent disparaître ou s'atténuer pour réapparaître ensuite sous une forme plus grave. L'évolution habituelle de la maladie est cyclique, entraînant une invalidité progressive, mais les formes cliniques sont très polymorphes. C'est une maladie invalidante dont le coût en suivi clinique, traitement symptomatique et prise en charge sont très élevés.

La SEP est une affection multifactorielle dans laquelle interviennent des facteurs liés à : l'environnement, une prédisposition génétique, et des désordres immunologiques. Le modèle qui prévaut se résume ainsi : un sujet prédisposé (grande fréquence des antigènes du système HLA : A3, B7, DR2DW2, DQB1 et DR4) rencontre un facteur exogène, vraisemblablement viral et/ou bactérien. Suit une longue phase de latence totalement silencieuse, au terme de laquelle se développent des réactions, pour une part autoimmunes, dirigées contre la myéline et la cellule productrice, l'oligodendrocyte. Ces désordres se traduisent par des lésions démyélinisantes du système nerveux central (SNC). La multiplicité des facteurs intervenant dans la SEP, et leur complexité, constituent des obstacles à la compréhension de son étiologie. C'est pourquoi, malgré les nombreux travaux effectués, celle-ci demeure toujours inconnue.

Les solutions suivantes ont été préparées

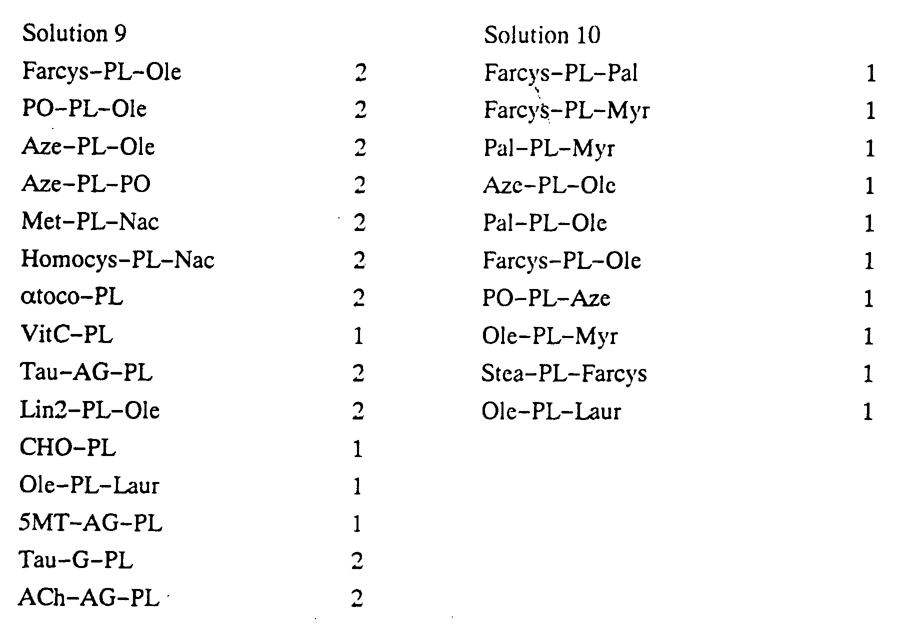

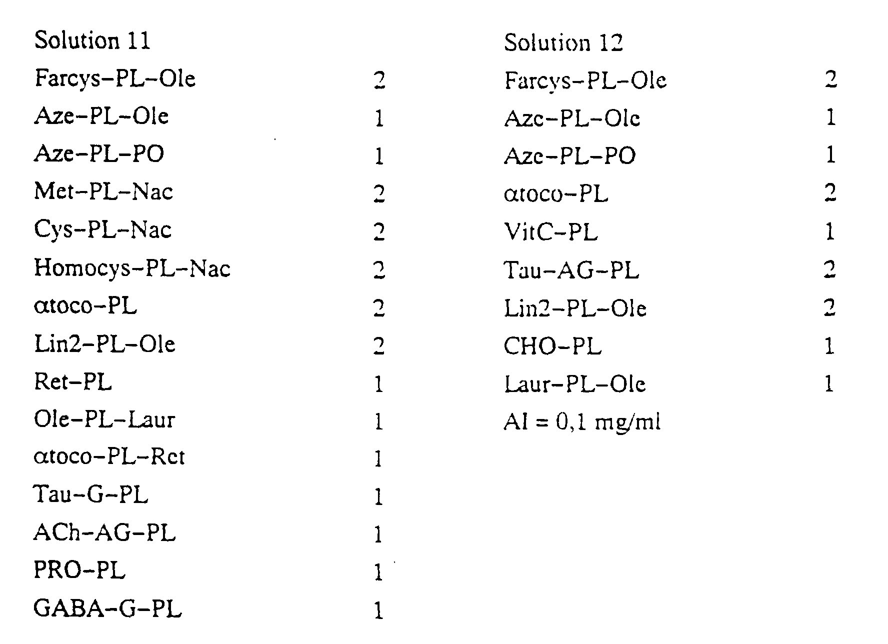

### Préparation: comme pour l'exemple 1.

Selon la forme clinique de la sclérose en plaques (SEP), l'administration (par injection sous-cutanée) des solutions 9, 10, 11 et 12 varic.
a) Dans le cas d'une poussée ou aggravation de la SEP, on utilise alternativement au moins deux des solutions 9, 10 ou 11, à raison de 0,5 ml à 1 ml de ces solutions une à deux fois par jour, ou bien de 0,5 ml à 1 ml de ces solutions tous les 2-3 jours, ou une fois par semaine. Par exemple, du 1er au 8ème jour de traitement, on administre alternativement, une ou deux fois par jour, les solutions 9 et 10. Du 8ème au 10ème jour de traitement, on administre alternativement, une fois par jour, les solutions 9 et 11. Du 10ème au 20ème jour de traitement, on administre alternativement, tous les deux jours, les solutions 9 et 11. Puis, après le 20ème jour de traitement, on utilise en alternance les solutions 9, 10 et 11 tous les 2-3 jours.
   Sur tous les cas (n = 15) de SEP en poussée ou aggravation traités selon le protocole ci-dessus depuis juin 1994, on a observé 100 % de régression de la symptomatologie.
b) Dans le cas de la maladie chronique ou de la récupération des séquelles, on administre alternativement, à raison de 0.5 ml à 0,8 ml par jour les solutions 9, 11 et 12. Par exemple, on administre une solution par jour, le matin ou le soir.

On note dans 80 % des cas (n = 250) traités une régression significative de la symptomatologie.

### Exemple 6 : Préparation d'une composition pharmaceutique pour le traitement des épilepsies

Les épilepsies essentielles peuvent revêtir différentes formes cliniques et atteintes électro-encéphalographiques, allant des absences au grand mal épileptique.

On a préparé la solution suivante :

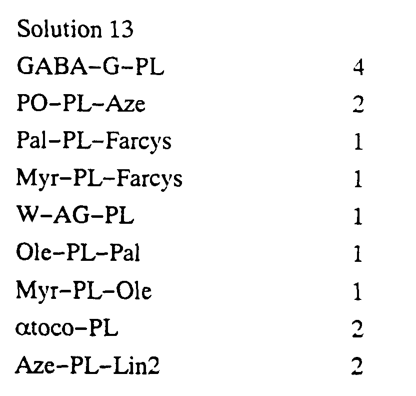

### Préparation: comme pour l'exemple 1.

La solution 13 est administrée par injection sous-cutanée, à raison de 0,6 ml de solution 2 à 3 fois par semaine.

Les deux malades traités ont diminué leurs médications classiques (Zarontin®, Dépamide®,....) sans inconvénient majeur.

### Exemple 7 : Préparation d'une composition pharmaceutique pour le traitement des migraines essentielles

Celles-ci n'ont pas de cause apparente. Elles se présentent avec ou sans aura. Elles sont liées à un réflexe vasogénique impliquant la sérotonine et ses dérivés.

On a préparé la solution suivante

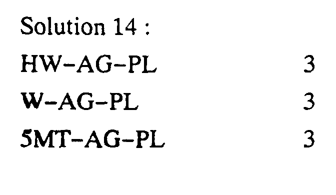

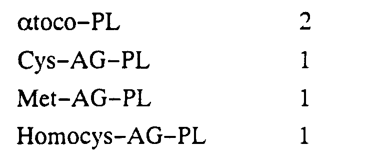

### Préparation : comme pour l'exemple 1.

La solution 14 est administrée par injection sous-cutanée, à raison de 0,8 ml de solution 2 à 3 fois par jour en cas de crise. En cas d'état migraineux permanent, on administre de préférence 0,8 ml de solution tous les 2 à 3 jours.

Sur trois cas traités, on observe un arrêt de la crise ou une atténuation de l'état migraineux.

### Exemple 8 : Préparation d'une association pharmaceutique pour le traitement des polyarthrites.

La polyarthrite rhumatoïde (PR) est une affection à caractère autoimmun dont l'étiologie demeure inconnue.

Les rhumatismes inflammatoires entraînent une atteinte importante des articulations avec déformations invalidantes et douleurs intenses.

On ne connaît pas de traitement spécifique pour la PR. Les principaux médicaments utilisés : sels d'or, D-penicillamine, sulfasalazine, méthotréxate, antimalariques, anti-inflammatoires non stéroïdiens, immunodépresseurs, immunostimulants, agissent soit sur l'immunité, soit sur l'inflammation. Mais, ces médicaments présentent des limites et des inconvénients:
- à court terme, ils ont une efficacité partielle et chez certains malades seulement ;
- à long terme, il n'existe aucune preuve de leur efficacité, sinon de nombreux effets secondaires (troubles gastro-intestinaux, immunodépression, etc...) ;
- ils ne sont pas anodins, entraînant parfois des accidents graves.

On a préparé les solutions suivantes :

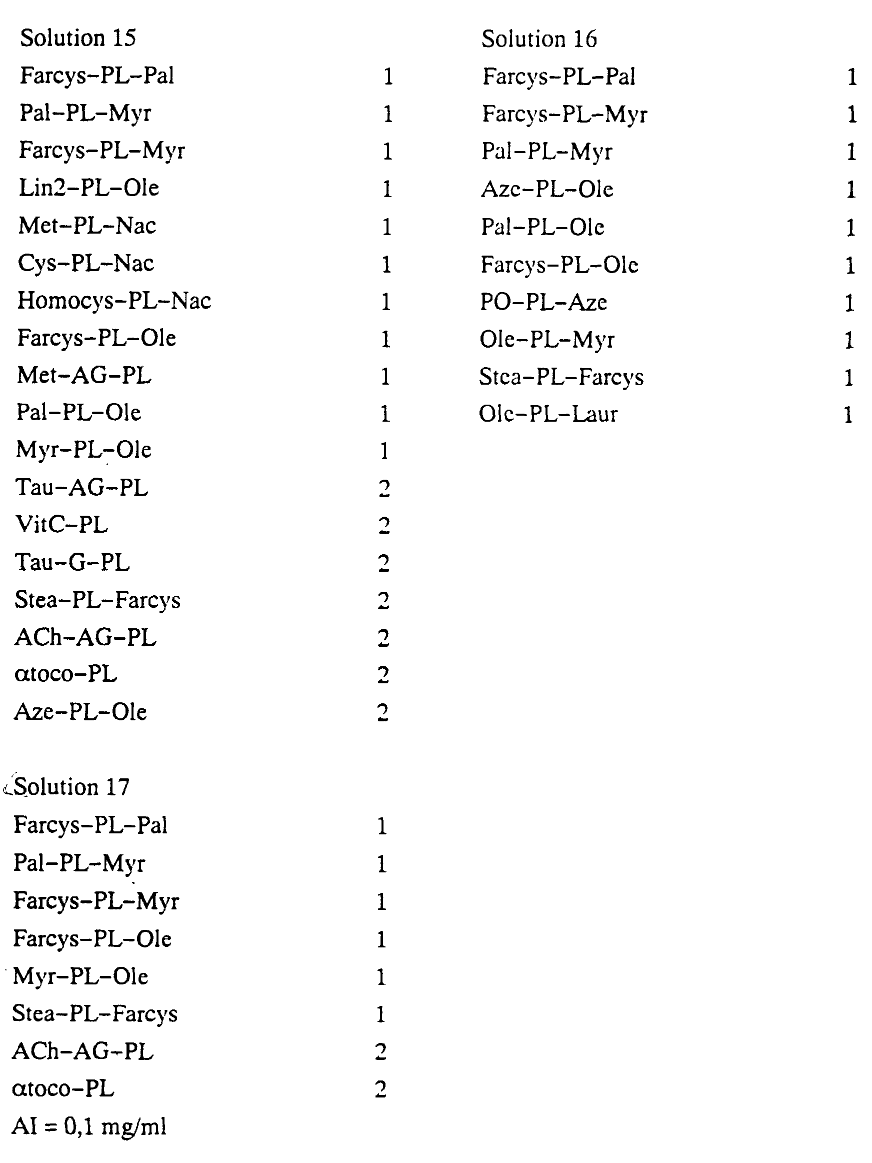

### Préparation: comme pour l'exemple 1.

Selon la forme clinique de la polyarthrite rhumatoïde, l'administration, par voie sous-cutanée, des solutions 15, 16 et 17 varie.
a) en période de poussée ou d'aggravation, on utilise les solutions 15 et 16, à raison d'un mélange de 0,1 ml à 0,5 ml de chaque solution, une à deux fois par jour.
b) en phase chronique, on utilise alternativement
   - soit les solutions 15 et 16 mélangées ensemble ;
   - soit les solutions 16 et 17 mélangées ensemble
à raison de 0,1 ml à 0,5 ml de chaque solution, tous les 2, 3 ou 4 jours ou toutes les semaines.

On constate chez les sujets (n = 150) traités une évolution favorable de la polyarthrite rhumatoïde dans 80 % des cas, même pour des affections anciennes rebelles aux autres thérapeutiques.

### Références bibliographiques

1. Daverat et al., J. Neuroimmunol. : 22, 129-134 (1989)
2. Amara et al., AIDS : 8, 711-713 (1994)
3. Maneta-Peyret et al., Neuroscience Letters : 80, 235-239 (1987)
4. Brochet et al., Current Concepts in Multiple Sclerosis : 10, 97-102 (1991)
5. Boullerne et al., Satellite Symposium to 17th ENA Meeting, Prague, Septembre 1994
6. Buttke et al., Immunology Today : 15, 7-10 (1994)
7. Hunter et al., Neurochem. Res. : 10, 1645-1659 (1985)
8. Korpela et al., J. Neurol. Sci. : 91, 79-84 (1989)
9. Roederer et al., Pharmacol. : 46, 121-129 (1993)
10. Staal et al., The Lancet : 339, 909-912 (1992)
11. Geffard et al., Science : 229, 77-79 (1985)
12. Souan et al., Neuroscience Letters : 64, 23-28 (1986)
13. Geffard et al., J. Neurochem. : 44, 1221-1228 (1985)
14. Geffard et al., Neurochem. Int. : 7, 403-413 (1985)
15. Seguela et al., Proc. Natl. Acad. Sci. USA : 81, 3888-3892 (1984)
16. Geffard et al., C.R. Acad. Sci. Paris : 295, 797-802 (1982)
17. Geffard et al., Brain Res. : 294, 161-165 (1984)
18. Chagnaud et al., Polycyclic Aromatic compounds : 663-672 (1993) (Ph. Garrigues and M. Lamotte Eds., Gordon and Breach Science Publishers)
19. Chagnaud et al., Polycyclic Aromatic compounds : 1119-1126 (1993) (Ph. Garrigues and M. Lamotte Eds., Gordon and Breach Science Publishers)
20. Chagnaud et al., Anti-cancer drugs : 5, 361-366 (1994)
21. Campistron et al., Brain Research : 365, 179-184 (1986)

## Revendications

1. Utilisation d'au moins un conjugué monofonctionnel et/ou polyfonctionnel entre d'une part la polylysine et d'autre part une ou plusieurs molécule(s) choisie(s) parmi:
a)
- les (di)acides gras à chaîne linéaire ou ramifiée, saturés ou insaturés, comprenant de 4 à 24 atomes de carbone ;
- les isoprénoïdes de 6 à 20 atomes de carbone liés à une cystéine ;
- le cholestérol et ses dérivés, notamment les hormones hydrophobes ;
b)
- la vitamine A, la vitamine C, la vitamine E ou un de leurs dérivés ;
- la cystéine et ses dérivés, de formule :
R₁S-R₂-CH(NH₂)-COOH
dans laquelle R₁ représente H ou CH₃ et R₂ représente un C₁-C₃ alkylène ;
c)
- les indolealkylamines;
- les catécholamines;
- les acides aminés de formule :
R₃-CH(NH₂)-COOH
dans laquelle R₃ représente l'hydrogène, un groupe imidazol-5-ylméthyle, un groupe carboxyméthyle ou un groupe aminopropyle ;
- les acides amino(C₁-C₅)alkylsulfoniques ou sulfiniques;
- la carnitine ou la carnosine;
- les diamines de formule :
₂HN-A-NH₂
dans laquelle A représente un (C₁-C₆)alkylène ou un groupe -(CH₂)ₘ-NH-B-(CH₂)ₚ- dans lequel m et p, indépendamment l'un de l'autre, sont des entiers allant de 1 à 5, et B représente rien ou un groupe -(CH₂)ₙ-NH-, n étant un entier allant de 1 à 5 ;
- l'acétylcholine;
- l'acide γ-aminobutyrique.
pour la préparation d'une composition ou association pharmaceutique utile dans le traitement de la dégénérescence neuronale et des neuropathies infectieuses, traumatiques et toxiques, des affections dégénératives à caractère autoimmun, des troubles neurodégénératifs consécutifs à des affections génétiques et des affections prolifératives,
étant entendu que les conjugués monofonctionnels entre la polylysine et:
- les (di)acide gras, à chaîne linéaire ou ramifiée, saturés ou insaturés, comprenant de 4 à 24 atomes de carbone ;
- les isoprénoïdes de 6 à 20 atomes de carbone liés à une cystéine ; ou
- le cholestérol et ses dérivés,
ne sont pas utilisés pour la préparation de médicaments destinés au traitement des infections à VIH.

2. Utilisation selon la revendication 1 pour la préparation d'une composition ou association pharmaceutique utile pour le traitement des troubles de la mémoire, démence vasculaire, troubles post-encéphalitiques, troubles post-apoplectiques, syndromes post-traumatiques dus à un traumatisme crânien, troubles dérivant d'anoxies cérébrales, maladie d'Alzheimer, démence sénile, démence subcorticale telle que la chorée de Huntington et la maladie de Parkinson, démence provoquée par le SIDA, neuropathies dérivées de morbidité ou dommage des nerfs sympathiques ou sensoriels, maladies cérébrales telles que l'oedème cérébral, dégénérescences spinocérébelleuses, neuropathies consécutives à une maladie de Lyme, affections présentant des troubles neurodégénératifs telles que la maladie de Charcot-Marie-Tooth, sclérose latérale amyotrophique, sclérose en plaques, épilepsies, migraines, polyarthrites, diabète insulino-dépendant, lupus érythémateux disséminé, thyroïdite de Hashimoto, maladie de Horton, dermatomyosite ou polymyosite, spondylarthrite ankylosante, infection à VIH ou cancers.

3. Utilisation selon la revendication 1 ou 2 d'au moins un conjugué entre la polylysine et une molécule a), au moins un conjugué entre la polylysine et une molécule b) et au moins un conjugué entre la polylysine et une molécule c).

4. Utilisation selon la revendication 1 ou la revendication 2, caractérisée en ce que ledit conjugué monofonctionnel est un conjugué entre la polylysine et un (di)acide gras.

5. Utilisation selon la revendication 1 ou la revendication 2, caractérisée en ce que ledit conjugué polyfonctionnel est un conjugué bifonctionnel et en ce que au moins une des molécules dudit conjugué bifonctionnel est un (di)acide gras.

6. Utilisation selon la revendication 4 ou la revendication 5, caractérisée en ce que le (di)acide gras est choisi parmi l'acide myristique, l'acide palmitique, l'acide stéarique, l'acide oléique ou l'acide azélaïque.

7. Conjugués monofonctionnels entre d'une part la polylysine et d'autre part une molécule choisie parmi :
a)
- une hormone hydrophobe ;
b)
- la vitamine A, la vitamine C, la vitamine E ou un de leurs dérivés ;
c)
- un acide aminé de formule :
R₃-CH(NH₂)-COOH
dans laquelle R₃ représente l'hydrogène, un groupe imidazol-5-ylméthyle ou un groupe aminopropyle ;
- un acide amino(C₁-C₅)alkylsulfonique ou sulfinique;
- la carnitine ou la carnosine ; ou
- une diamine de formule:
₂HN-A-NH₂
dans laquelle A représente un (C₁-C₆)alkylène ou un groupe -(CH₂)ₘ-NH-B-(CH₂)ₚ- dans lequel m et p, indépendamment l'un de l'autre, sont des entiers allant de 1 à 5, et B représente rien ou un groupe -(CH₂)ₙ-NH-, n étant un entier allant de 1 à 5.

8. Conjugués polyfonctionnels entre d'une part au moins deux molécules choisies parmi :
a)
- les (di)acides gras à chaîne linéaire ou ramifiée, saturés ou insaturés, comprenant de 4 à 24 atomes de carbone ;
- les isoprénoïdes de 6 à 20 atomes de carbone liés à une cystéine ;
- le cholestérol et ses dérivés, notamment les hormones hydrophobes ;
b)
- la vitamine A, la vitamine C, la vitamine E ou un de leurs dérivés ;
- la cystéine et ses dérivés, de formule
R₁S-R₂-CH(NH₂)-COOH
dans laquelle R₁ représente H ou CH₃ et R₂ représente un C₁-C₃ alkylène ;
c)
- les indolealkylamines;
- les catécholamines;
- les acides aminés de formule :
R₃-CH(NH₂)-COOH
dans laquelle R₃ représente l'hydrogène, un groupe imidazol-5-ylméthyle, un groupe carboxyméthyle ou un groupe aminopropyle ;
- les acides amino(C₁-C₅)alkylsulfoniques ou sulfiniques;
- la carnitine ou la carnosine;
- les diamines de formule :
₂HN-A-NH₂
dans laquelle A représente un (C₁-C₆)alkylène ou un groupe -(CH₂)ₘ-NH-B-(CH₂)ₚ- dans lequel m et p, indépendamment l'un de l'autre, sont des entiers allant de 1 à 5, et B représente rien ou un groupe -(CH₂)ₙ-NH-, n étant un entier allant de 1 à 5 ;
- l'acétylcholine ; ou
- l'acide γ-aminobutyrique.
et d'autre part au moins une molécule de polylysine.

9. Conjugués selon la revendication 8, qui sont des conjugués bifonctionnels.

10. Compositions ou associations pharmaceutiques, qui contiennent au moins un conjugué tel que défini dans l'une quelconque des revendications 7 à 9.

11. Compositions ou associations selon la revendication 10, qui contiennent au moins un conjugué entre la polylysine et une molécule a), au moins un conjugué entre la polylysine et une molécule b), et au moins un conjugué entre la polylysine et une molécule c).

12. Composition pharmaceutique, choisie parmi:
* une composition qui comprend les conjugués suivants : L-DOPA-G-PL, αtoco-PL, Ole-PL-Farcys, PO-PL-Ole, Myr-PL-Ole, Cys-AG-PL ou Cys-AS-PL, Homocys-AG-PL ou Homocys-AS-PL, Met-AG-PL ou Met-AS-PL, Met-G-PL, Cys-G-PL, Homocys-G-PL, Laur-PL-Ole, GABA-AG-PL, 5MT-AG-PL, His-PL, Cys-PL-Nac, Homocys-PL-Nac, Met-PL-Nac,
* une composition qui comprend les conjugués suivants : GABA-G-PL, PO-PL-Aze, Pal-PL-Farcys, Myr-PL-Farcys, W-AG-PL, Ole-PL-Pal, Myr-PL-Ole, αtoco-PL, Aze-PL-Lin2, et
* une composition qui comprend les conjugués suivants : HW-AG-PL, W-AG-PL, MT-AG-PL, αtoco-PL, Cys-AG-PL, Met-AG-PL, Homocys-AG-PL

13. Association pharmaceutique, choisie parmi :
* une association qui comprend dans deux conditionnements distincts, d'une part les conjugués suivants (1a) : Farcys-PL-Pal, Pal-PL-Myr, Farcys-PL-Myr, Lin2-PL-Ole, Met-PL-Nac, Cys-PL-Nac, Homocys-PL-Nac, Farcys-PL-Ole, Met-AG-PL, Pal-PL-Ole, Myr-PL-Ole, Tau-AG-PL, VitC-PL, Tau-G-PL, Stea-PL-Farcys, ACh-AG-PL, αtoco-PL, Aze-PL-Ole ; et d'autre part les conjugués suivants (1b): Farcys-PL-Pal, Farcys-PL-Myr, Pal-PL-Myr, Aze-PL-Ole, Pal-PL-Ole, Farcys-PL-Ole, PO-PL-Aze, Ole-PL-Myr, Stea-PL-Farcys, Ole-PL-Laur,
* une association qui comprend dans deux conditionnements distincts, d'une part les conjugués suivants (2a) : Myr-PL-Pal, Pal-PL-Ole, Myr-PL-Ole, Tau-AS-PL ou Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL ou Met-AG-PL, Homocys-AS-PL ou Homocys-AG-PL, Cys-G-PL, Homocys-G-PL, Met-G-PL, CHO-PL, 5MT-AG-PL, W-AG-PL, ACh-AG-PL ; et d'autre part les conjugués suivants (2b) : Pal-PL-Ole, Tau-AS-PL ou Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL ou Met-AG-PL, Homocys-AS-PL ou Homocys-AG-PL, Cys-AG-PL, Cys-G-PL, Met-G-PL, Homocys-G-PL, Met-G-PL, CHO-PL, W-AG-PL, ACh-AG-PL, AI,
* une association qui comprend dans trois conditionnements distincts :
3a) les conjugués suivants : Myr-PL-Pal, Pal-PL-Ole, Myr-PL-Ole, Tau-AS-PL ou Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL ou Met-AG-PL, Homocys-AS-PL ou Homocys-AG-PL, Cys-G-PL, Homocys-G-PL, Met-G-PL, CHO-PL, 5MT-AG-PL, W-AG-PL, ACh-AG-PL ;
3b) les conjugués suivants : Cys-AG-PL, Met-AG-PL, Homocys-G-PL, Cys-PL-Nac, Homocys-PL-Nac, Met-PL-Nac, Tau-AG-PL, Tau-G-PL, Met-G-PL, αtoco-PL, αtoco-PL-Ret ;
et 3c) les conjugués suivants : Farcys-PL-Ole, Aze-PL-Ole, Aze-PL-PO, Met-PL-Nac, Cys-PL-Nac, Homocys-PL-Nac, αtoco-PL, Lin2-PL-Ole, Ret-PL, Ole-PL-Laur, αtoco-PL-Ret, Tau-G-PL, ACh-AG-PL, PRO-PL, GABA-G-PL,
* une association qui comprend dans quatre conditionnements distincts :
4a) les conjugués suivants : Farcys-PL-Ole, PO-PL-Ole, Aze-PL-Ole, Aze-PL-PO, Met-PL-Nac, Homocys-PL-Nac, αtoco-PL, VitC-PL, Tau-AG-PL, Lin2-PL-Ole, CHO-PL, Ole-PL-Laur, 5-MT-AG-PL, Tau-G-PL, ACh-AG-PL ;
4b) les conjugués suivants : Farcys-PL-Pal, Farcys-PL-Myr, Pal-PL-Myr, Aze-PL-Ole, Pal-PL-Ole, Farcys-PL-Ole, PO-PL-Aze, Ole-PL-Myr, Stea-PL-Farcys, Ole-PL-Laur ;
4c) les conjugués suivants : Farcys-PL-Ole, Aze-PL-Ole, Aze-PL-PO, Met-PL-Nac, Cys-PL-Nac, Homocys-PL-Nac, αtoco-PL, Lin2-PL-Ole, Ret-PL, Ole-PL-Laur, αtoco-PL-Ret, Tau-G-PL, ACh-AG-PL, PRO-PL, GABA-G-PL ;
et 4d) les conjugués suivants : Farcys-PL-Ole, Aze-PL-Ole, Aze-PL-PO, αtoco-PL, VitC-PL, Tau-AG-PL, Lin2-PL-Ole, CHO-PL, Laur-PL-Ole, AI,
* une association qui comprend dans trois conditionnements distincts :
5a) les conjugués suivants : Farcys-PL-Pal, Pal-PL-Myr, Farcys-PL-Myr, Lin2-PL-Ole, Met-PL-Nac, Cys-PL-Nac, Homocys-PL-Nac, Farcys-PL-Ole, Met-AG-PL, Pal-PL-Ole, Myr-PL-Ole, Tau-AG-PL, VitC-PL, Tau-G-PL, Stea-PL-Farcys, ACh-AG-PL, αtoco-PL, Aze-PL-Ole ;
5b) les conjugués suivants : Farcys-PL-Pal, Farcys-PL-Myr, Pal-PL-Myr, Aze-PL-Ole, Pal-PL-Ole, Farcys-PL-Ole, PO-PL-Aze, Ole-PL-Myr, Stea-PL-Farcys, Ole-PL-Laur ;
et 5c) les conjugués suivants : Farcys-PL-Pal, Pal-PL-Myr, Farcys-PL-Myr, Farcys-PL-Ole, Myr-PL-Ole, Stea-PL-Farcys, ACh-AG-PL, αtoco-PL, AI,
* une association qui comprend dans deux conditionnements distincts, d'une part les conjugués suivants (6a) : Lin2-PL-Farcys, Stea-PL-Farcys, Myr-PL-Farcys, Pal-PL-Farcys, Ole-PL-Farcys, Cys-AG-PL, Met-AG-PL, Homocys-AG-PL, CHO-PL, Homocys-PL-Nac, Cys-PL-Nac, αtoco-PL-Ret, αtoco-PL, VitC-PL, Ole-PL-Laur, Met-G-PL, Met-PL-Nac, Homocys-PL-Nac, Cys-G-PL; et d'autre part les conjugués mentionnés au point 3c) ci-dessus, et
* une association qui comprend dans trois conditionnements distincts:
7a) les conjugués suivants : PRO-PL, Ret-PL, αtoco-PL, αtoco-PL-Ret, W-AG-PL, SMT-AG-PL, Hist-PL;
7b) les conjugués mentionnés au point 1b) ci-dessus; et
7c) les conjugués suivants : L-DOPA-G-PL, L-DOPA-AG-PL, SMT-AG-PL, HW-AG-PL, Tyr-AG-PL.

14. Complément alimentaire ou vitaminé, qui comprend au moins un conjugué tel que défini dans l'une des revendications 7 à 9.

## Claims

1. Use of at least one monofunctional and/or polyfunctional conjugate between on the one hand polylysine and on the other hand one or more molecules selected from:
a)
- saturated or unsaturated, linear or branched fatty (di)acids comprising from 4 to 24 carbon atoms;
- isoprenoids having 6 to 20 carbon atoms and bonded to a cysteine; and
- cholesterol and its derivatives, especially hydrophobic hormones;
b)
- vitamin A, vitamin C, vitamin E or one of their derivatives; and
- cysteine and its derivatives of the formula
R₁S-R₂-CH(NH₂)-COOH
in which R₁ is H or CH₃ and R₂ is a C₁-C₃-alkylene; and
c)
- indolealkylamines;
- catecholamines;
- amino acids of the formula
R₃-CH(NH₂)-COOH
in which R₃ is hydrogen, an imidazol-5-ylmethyl group, a carboxymethyl group or an aminopropyl group;
- amino(C₁-C₅)alkylsulfonic or sulfinic acids;
- camitine or carnosine;
- diamines of the formula
H₂N-A-NH₂
in which A is a (C₁-C₆)alkylene or a group -(CH₂)ₘ-NH-B-(CH₂)ₚ-, in which m and p independently of one another are integers ranging from 1 to 5 and B is nothing or a group -(CH₂)ₙ-NH-, n being an integer ranging from 1 to 5;
- acetylcholine; and
- γ-aminobutyric acid,
for the preparation of a pharmaceutical composition or combination which is useful in the treatment of neuronal degeneration, infectious, traumatic and toxic neuropathies, degenerative diseases of the autoimmune type, neurodegenerative disorders resulting from genetic diseases, and proliferative diseases, it being understood that the monofunctional conjugates between polylysine and:
- saturated or unsaturated, linear or branched fatty (di)acids comprising from 4 to 24 carbon atoms;
- isoprenoids having 6 to 20 carbon atoms and bonded to a cysteine; or
- cholesterol and its derivatives,
are not used for the preparation of drugs intended for the treatment of HIV infections.

2. Use according to claim 1 for the preparation of a pharmaceutical composition or combination which is useful for the treatment of memory disorders, vascular dementia, postencephalitic disorders, postapoplectic disorders, post-traumatic syndromes due to a cranial traumatism, disorders derived from cerebral anoxia, Alzheimer's disease, senile dementia, subcortical dementia such as Huntington's chorea and Parkinson's disease, dementia caused by AIDS, neuropathies derived from morbidity or damage to the sympathetic or sensory nerves, brain diseases such as cerebral edema, spinocerebellar degenerations, neuropathies resulting from Lyme disease, diseases presenting neurodegenerative disorders, such as Charcot-Marie-Tooth disease, amyotrophic lateral sclerosis, multiple sclerosis, epilepsy, migraine, polyarthritis, insulin-dependent diabetes, systemic lupus erythematosus, Hashimoto's thyroiditis, Horton's disease, dermatomyositis or polymyositis, ankylosing spondylarthritis, HIV infection or cancer.

3. Use according to claim 1 or 2 of at least one conjugate between polylysine and a molecule a), at least one conjugate between polylysine and a molecule b) and at least one conjugate between polylysine and a molecule c).

4. Use according to claim 1 or claim 2, characterized in that said monofunctional conjugate is a conjugate between polylysine and a fatty (di)acid.

5. Use according to claim 1 or claim 2, characterized in that said polyfunctional conjugate is a bifunctional conjugate and in that at least one of the molecules of said bifunctional conjugate is a fatty (di)acid.

6. Use according to claim 4 or claim 5, characterized in that the fatty (di)acid is selected from myristic acid, palmitic acid, stearic acid, oleic acid and azelaic acid.

7. Monofunctional conjugates between on the one hand polylysine and on the other hand a molecule selected from:
a)
- a hydrophobic hormone;
b)
- vitamin A, vitamin C, vitamin E or one of their derivatives;
c)
- an amino acid of the formula
R₃-CH(NH₂)-COOH
in which R₃ is hydrogen, an imidazol-5-ylmethyl group or an aminopropyl group;
- an amino(C₁-C₅)alkylsulfonic or sulfinic acid;
- carnitine or carnosine; and
- a diamine of the formula
H₂N-A-NH₂
in which A is a (C₁-C₆)alkylene or a group -(CH₂)ₘ-NH-B-(CH₂)ₚ-, in which m and p independently of one another are integers ranging from 1 to 5 and B is nothing or a group -(CH₂)ₙ-NH-, n being an integer ranging from 1 to 5.

8. Polyfunctional conjugates between on the one hand at least two molecules selected from:
a)
- saturated or unsaturated, linear or branched fatty (di)acids comprising from 4 to 24 carbon atoms;
- isoprenoids having 6 to 20 carbon atoms and bonded to a cysteine; and
- cholesterol and its derivatives, especially hydrophobic hormones;
b)
- vitamin A, vitamin C, vitamin E or one of their derivatives; and
- cysteine and its derivatives of the formula
R₁S-R₂-CH(NH₂)-COOH
in which R₁ is H or CH₃ and R₂ is a C₁-C₃-alkylene; and
c)
- indolealkylamines;
- catecholamines;
- amino acids of the formula
R₃-CH(NH₂)-COOH
in which R₃ is hydrogen, an imidazol-5-ylmethyl group, a carboxymethyl group or an aminopropyl group;
- amino(C₁-C₅)alkylsulfonic or sulfinic acids;
- carnitine or carnosine;
- diamines of the formula
H₂N-A-NH₂
in which A is a (C₁-C₆)alkylene or a group -(CH₂)ₘ-NH-B-(CH₂)ₚ-, in which m and p independently of one another are integers ranging from 1 to 5 and B is nothing or a group -(CH₂)ₙ-NH-, n being an integer ranging from 1 to 5;
- acetylcholine; and
- γ-aminobutyric acid,
and on the other hand at least one polylysine molecule.

9. Conjugates according to claim 8 which are bifunctional conjugates.

10. Pharmaceutical compositions or combinations which contain at least one conjugate as defined in any one of claims 7 to 9.

11. Compositions or combinations according to claim 10 which contain at least one conjugate between polylysine and a molecule a), at least one conjugate between polylysine and a molecule b) and at least one conjugate between polylysine and a molecule c).

12. A pharmaceutical composition, which is selected from
* a composition comprising the following conjugates : L-DOPA-G-PL, αtoco-PL, Ole-PL-Farcys, PO-PL-Ole, Myr-PL-Ole, Cys-AG-PL or Cys-AS-PL, Homocys-AG-PL or Homocys-AS-PL, Met-AG-PL or Met-AS-PL, Met-G-PL, Cys-G-PL, Homocys-G-PL, Laur-PL-Ole, GABA-AG-PL, 5MT-AG-PL, His-PL, Cys-PL-Nac, Homocys-PL-Nac, Met-PL-Nac ;
* a composition comprising the following conjugates : GABA-G-PL, PO-PL-Aze, Pal-PL-Farcys, Myr-PL-Farcys, W-AG-PL, Ole-PL-Pal, Myr-PL-Ole, αtoco-PL, Aze-FL-Lin2 ; and
* a composition comprising the following conjugates : HW-AG-PL, W-AG-PL, MT-AG-PL, αtoco-PL, Cys-AG-PL, Met-AG-PL, Homocys-AG-PL.

13. A pharmaceutical combination which is selected from :
* a combination comprising in two separate packs, on the one hand the following conjugate (1a) : Farcys-PL-Pal, Pal-PL-Myr, Farcys-PL-Myr, Lin2-PL-Ole, Met-PL-Nac, Cys-FL-Nac, Homocys-PL-Nac, Farcys-PL-Ole, Met-AG-PL, Pal-PL-Ole, Myr-PL-Ole, Tau-AG-PL, VitC-PL, Tau-G-PL, Stea-PL-Farcys, Ach-AG-PL, αtoco-PL, Aze-PL-Ole ; and on the other hand the following conjugates (1b): Farcys-PL-Pal, FarCys-PL-Myr, Pal-PL-Myr, Aze-PL-Ole, Pal-PL-Ole, Farcys-PL-Ole, PO-PL-Aze, Ole-PL-Myr, Stea-PL-Farcys, Ole-PL-Laur ;
* a combination comprising in two separate packs, on the one hand the following conjugates (2a) : Myr-PL-Pal, Pal-PL-Ole, Myr-PL-Ole, Tau-AS-PL or Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL or Met-AG-PL, Homocys-AS-PL or Homocys-AG-PL, Cys-G-PL, Homocys-G-PL, Met-G-PL, CHO-PL, 5MT-AG-PL, W-AG-PL, Ach-AG-PL ; and on the other hand the following conjugates (2b) : Pal-PL-Ole, Tau-AS-PL or Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL or Met-AG-PL, Homocys-AS-PL or Homocys-AG-PL, Cys-AG-PL, Cys-G-PL, Met-G-PL, Homocys-G-PL, Met-G-PL, CHO-PL, W-AG-PL, Ach-AG-Pl, AI ;
* a combination comprising in three separate packs :
3a) the following conjugates : Myr-PL-Pal, Pal-PL-Ole, Myr-PL-Ole, Tau-AS-PL or Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL or Met-AG-PL, Homocys-AS-PL or Homocys-AG-PL, Cys-G-PL, Homocys-G-PL, Met-G-PL, CHO-PL, 5MT-AG-PL, W-AG-PL, ACh-AG-PL ;
3b) the following conjugates : Cys-AG-PL, Met-AG-PL, Homocys-G-PL, Cys-PL-Nac, Homocys-PL-Nac, Met-PL-Nac, Tau-AG-PL, Tau-G-PL, Met-G-PL, αtoco-PL, αtoco-PL-Ret ;
and 3c) the following conjugates : Farcys-PL-Ole, Aze-PL-Ole, Aze-PL-PO, Met-PL-Nac, Cys-PL-Nac, Homocys-PL-Nac, αtoco-PL, Lin2-PL-Ole, Ret-PL, Ole-PL-Laur, αtoco-PL-Ret, Tau-G-PL, ACh-AG-PL, PRO-PL, GABA-G-PL ;
* a combination comprising in four separate packs :
4a) the following conjugates : Farcys-PL-Ole, PO-PL-Ole, Aze-PL-Ole, Aze-PL-PO, Met-PL-Nac, Homocys-PL-Nac, αtoco-PL, VitC-PL, Tau-AG-PL, Lin2-PL-Ole, CHO-PL, Ole-PL-Laur, 5-MT-AG-PL, Tau-G-PL, ACh-AG-PL ;
4b) the following conjugates : Farcys-PL-Pal, Farcys-PL-Myr, Pal-PL-Myr, Aze-PL-Ole, Farcys-PL-Ole, Pal-PL-Ole, PO-PL-Aze, Ole-PL-Myr, Stea-PL-Farcys, Ole-PL-Laur ;
4c) the following conjugates : Farcys-PL-Ole, Aze-PL-Ole, Aze-PL-PO, Met-PL-Nac, Cys-PL-Nac, Homocys-PL-Nac, αtoco-PL, Lin2-PL-Ole, Ret-PL, Ole-PL-Laur, αtoco-PL-Ret, Tau-G-PL, ACh-AG-PL, PRO-PL, GABA-G-PL ;
and 4d) the following conjugates : Farcys-PL-Ole, Aze-PL-Ole, Aze-PL-PO, αtoco-PL, VitC-PL, Tau-AG-PL, Lin2-PL-Ole, CHO-PL, Laur-PL-Ole, AI ;
* a combination comprising in three separate packs :
5a) the following conjugates : Farcys-PL-Pal, Pal-PL-Myr, Farcys-PL-Myr, Lin2-PL-Ole, Met-PL-Nac, Cys-PL-Nac, Homocys-PL-Nac, Farcys-PL-Ole, Met-AG-PL, Pat-PL-Ole, Myr-PL-Ole, Tau-AG-PL, VitC-PL, Tau-G-PL, Stea-PL-Farcys, ACh-AG-PL, αtoco-PL, Aze-PL-Ole ;
5b) the following conjugates : Farcys-PL-Pal, Farcys-PL-Myr, Pal-PL-Myr, Aze-PL-Ole, Pal-PL-Ole, Farcys-PL-Ole, PO-PL-Aze, Ole-PL-Myr, Stea-PL-Farcys, Ole-PL-Laur ;
and 5c) the following conjugates : Farcys-PL-Pal, Pal-PL-Myr, Farcys-PL-Myr, Farcys-PL-Ole, Myr-PL-Ole, Stea-PL-Farcys, ACh-AG-PL, αtoco-PL, AI ;
* a combination comprising in two separate packs, on the one hand the following conjugates (6a) : Lin2-PL-Farcys, Stea-PL-Farcys, Myr-PL-Farcys, Pal-PL-Farcys, Ole-PL-Farcys, Cys-AG-PL, Met-AG-PL, Homocys-AG-PL, CHO-PL, Homocys-PL-Nac, Cys-PL-Nac, αtoco-PL-Ret, αtoco-PL, VitC-PL, Ole-PL-Laur, Met-G-PL, Met-PL-Nac, Homocys-PL-Nac, Cys-G-PL ; and on the other hand the conjugates mentioned in point 3c) above ; and
* a combination comprising in three separate packs:
7a) the following conjugates : PRO-PL, Ret-PL, αtoco-PL, αtoco-PL-Ret, W-AG-PL, 5MT-AG-PL, Hist-PL ;
7b) the conjugates mentioned in point 1b) above ; and
7c) the following conjugates : L-DOPA-G-PL, L-DOPA-AG-PL, 5MT-AG-PL, HW-AG-PL, Tyr-AG-PL.

14. Food or vitamin supplement, which comprises at least one conjugate as defined in one of claims 7 to 9.

## Patentansprüche

1. Verwendung mindestens eines monofunktionellen und/oder polyfunktionellen Konjugats aus einerseits Polylysin und andererseits einem oder mehr Molekül(en) ausgewählt aus:
a)
- gesättigten oder ungesättigten (Di-) Fettsäuren mit gerader oder verzweigter Kette und 4 bis 24 Kohlenstoffatomen;
- an ein Cystein gebundenen Isoprenoiden mit 6 bis 20 Kohlenstoffatomen;
- Cholesterin und Derivaten desselben, insbesondere hydrophoben Hormonen;
b)
- Vitamin A, Vitamin C, Vitamin E oder einem Derivat derselben;
- Cystein und Derivaten desselben der Formel:
R₁S-R₂-CH(NH₂)-COOH
worin R₁ H oder CH₃ darstellt und R₂ C₁-C₃-Alkylen darstellt;
c)
- Indolalkylaminen;
- Catecholaminen;
- Aminosäuren der Formel
R₃-CH(NH₂)-COOH
worin R₃ Wasserstoff, Imidazol-5-yl-methyl, Carboxymethyl oder Aminopropyl darstellt;
- Amino-(C₁-C₅)-Alkylsulfon- oder -sulfinsäuren;
- Carnitin oder Carnosin;
- Diaminen der Formel
₂HN-A-NH₂
worin A (C₁-C₆)-Alkylen oder - (CH₂)ₘ-NH-B-(CH₂)ₚ- darstellt, worin m und p unabhängig voneinander ganze Zahlen von 1 bis 5 sind und B nichts oder -(CH₂)ₙ-NH- darstellt, wobei n eine ganze Zahl von 1 bis 5 ist;
- Acetylcholin;
- γ-Aminobuttersäure;
zur Herstellung einer pharmazeutischen Zusammensetzung oder Assoziation, die geeignet ist zur Behandlung von neuronaler Degenerierung und infektiösen, traumatischen und toxischen Neuropathien, degenerativen Erkrankungen mit automimmunem Charakter, neurodegenerativen Störungen infolge genetischer Erkrankungen und proliferativer Erkrankungen,
mit der Maßgabe, dass die monofunktionellen Konjugate aus Polylysin und
- gesättigten oder ungesättigten (Di-) Fettsäuren mit gerader oder verzweigter Kette und 4 bis 24 Kohlenstoffatomen,
- an ein Cystein gebundenen Isoprenoiden mit 6 bis 20 Kohlenstoffatomen, oder
- Cholesterin und Derivaten desselben
nicht zur Herstellung von Medikamenten verwendet werden, die zur Behandlung von HIV-Infektionen bestimmt sind.

2. Verwendung nach Anspruch 1 zur Herstellung einer pharmazeutischen Zusammensetzung oder Assoziation, die geeignet ist zur Behandlung von Gedächtnisstörungen, vaskulärer Demenz, postenzephalitischen Störungen, postapoplektischen Störungen, posttraumatischen Syndromen aufgrund eines Schädeltraumas, Störungen, die auf zerebrale Anoxien zurückzuführen sind, Alzheimer Krankheit, seniler Demenz, subkortikaler Demenz wie Chorea Huntington und Parkinsonscher Krankheit, durch AIDS hervorgerufener Demenz, Neuropathien, die auf eine Krankhaftigkeit oder einen Schaden des sympathischen oder sensorischen Nervensystems zurückzuführen sind, zerebralen Erkrankungen wie einem zerebralen Ödem, spinozerebellaren Degenerationen, Neuropathien infolge einer Lyme-Krankheit, Erkrankungen mit neurodegenerativen Störungen wie der Charcot-Marie-Tooth-Krankheit, amyotropher Lateralsklerose, Plaque-Sklerose, Epilepsie, Migräne, Polyarthritis, Insulin-abhängigem Diabetes, Lupus erythematodes disseminatus, Hashimoto Thyreoiditis, Horton-Krankheit, Dermatomyositis oder Polymyositis, Spondylarthritis ankylosans, HIV-Infektionen oder Krebserkrankungen.

3. Verwendung nach Anspruch 1 oder 2, mindestens eines Konjugats aus Polylysin und einem Molekül a), mindestens eines Konjugats aus Polylysin und einem Molekül b) und mindestens eines Konjugats aus Polylysin und einem Molekül c).

4. Verwendung nach Anspruch 1 oder 2, dadurch gekennzeichnet, dass das monofunktionelle Konjugat ein Konjugat aus Polylysin und einer (Di-) Fettsäure ist.

5. Verwendung nach Anspruch 1 oder 2, dadurch gekennzeichnet, dass das polyfunktionelle Konjugat ein bifunktionelles Konjugat ist und dass mindestens eines der Moleküle des bifunktionellen Konjugats eine (Di-) Fettsäure ist.

6. Verwendung nach Anspruch 4 oder 5, dadurch gekennzeichnet, dass die (Di-) Fettsäure ausgewählt ist aus Myristinsäure, Palmitinsäure, Stearinsäure, Oleinsäure oder Azelainsäure.

7. Monofunktionelle Konjugate aus einerseits Polylysin und andererseits einem Molekül ausgewählt aus:
a)
- einem hydrophoben Hormon;
b)
- Vitamin A, Vitamin C, Vitamin E oder einem Derivat derselben;
c)
- einer Aminosäure der Formel
R₃-CH(NH₂)-COOH
worin R₃ Wasserstoff, Imidazol-5-yl-methyl oder Aminopropyl darstellt;
- einer Amino-(C₁-C₅)-Alkylsulfon- oder -sulfinsäure;
- Carnitin oder Carnosin; oder
- einem Diamin der Formel
₂HN-A-NH₂
worin A (C₁-C₆)-Alkylen oder -(CH₂)ₘ-NH-B-(CH₂)ₚ- darstellt, worin m und p unabhängig voneinander ganze Zahlen von 1 bis 5 sind und B nichts oder -(CH₂)ₙ-NH- darstellt, wobei n eine ganze Zahl von 1 bis 5 ist.

8. Polyfunktionelle Konjugate aus einerseits mindestens zwei Molekülen ausgewählt aus:
a)
- gesättigten oder ungesättigten (Di-) Fettsäuren mit gerader oder verzweigter Kette und 4 bis 24 Kohlenstoffatomen;
- an ein Cystein gebundenen Isoprenoiden mit 6 bis 20 Kohlenstoffatomen;
- Cholesterin und Derivaten desselben, insbesondere hydrophoben Hormonen;
b)
- Vitamin A, Vitamin C, Vitamin E oder einem Derivat derselben;
- Cystein und Derivaten desselben der Formel:
R₁S-R₂-CH(NH₂)-COOH
worin R₁ H oder CH₃ darstellt und R₂ C₁-C₃-Alkylen darstellt;
c)
- Indolalkylaminen;
- Catecholaminen;
- Aminosäuren der Formel
R₃-CH(NH₂)-COOH
worin R₃ Wasserstoff, Imidazol-5-yl-methyl, Carboxymethyl oder Aminopropyl darstellt;
- Amino-(C₁-C₅)-Alkylsulfon- oder -sulfinsäuren;
- Carnitin oder Carnosin;
- Diaminen der Formel
₂HN-A-NH₂
worin A (C₁-C₆)-Alkylen oder - (CH₂)ₘ-NH-B-(CH₂)ₚ- darstellt, worin m und p unabhängig voneinander ganze Zahlen von 1 bis 5 sind und B nichts oder -(CH₂)ₙ-NH- darstellt, wobei n eine ganze Zahl von 1 bis 5 ist;
- Acetylcholin oder
- γ-Aminobuttersäure
und andererseits mindestens einem Polylysinmolekül.

9. Konjugate nach Anspruch 8, welche bifunktionelle Konjugate sind.

10. Pharmazeutische Zusammensetzungen oder Assoziationen, die mindestens ein Konjugat wie in einem der Ansprüche 7 bis 9 definiert enthalten.

11. Zusammensetzungen oder Assoziationen nach Anspruch 10, die mindestens ein Konjugat aus Polylysin und einem Molekül a), mindestens ein Konjugat aus Polylysin und einem Molekül b) und mindestens ein Konjugat aus Polylysin und einem Molekül c) enthalten.

12. Pharmazeutische Zusammensetzung ausgewählt aus:
* einer Zusammensetzung, die folgende Konjugate enthält:
L-DOPA-G-PL, αtoco-PL, Ole-PL-Farcys, PO-PL-Ole, Myr-PL-Ole, Cys-AG-PL oder Cys-AS-PL, Homocys-AG-PL oder Homocys-AS-PL, Met-AG-PL oder Met-AS -PL, Met-G-PL, Cys-G-PL, Homocys-G-PL, Laur-PL-Ole, GABA-AG-PL, 5MT-AG-PL, His-PL, Cys-PL-Nac, Homo-cys-PL-Nac, Met-PL-Nac,
* einer Zusammensetzung, die folgende Konjugate enthält:
GABA-G-PL, PO-PL-Aze, Pal-PL-Farcys, Myr-PL-Farcys, W-AG-PL, Ole-PL-Pal, Myr-PL-Ole, αtoco-PL, Aze-PL-Lin2, und
* einer Zusammensetzung, die folgende Konjugate enthält:
HW-AG-PL, W-AG-PL, MT-AG-PL, αtoco-PL, Cys-AG-PL, Met-AG-PL, Homocys-AG-PL.

13. Pharmazeutische Assoziation ausgewählt aus:
* einer Assoziation, die in zwei unterschiedlichen Packungen einerseits die folgenden Konjugate (1a):
Farcys-PL-Pal, Pal-PL-Myr, Farcys-PL-Myr, Lin2-PL-Ole, Met-PL-Nac, Cys-PL-Nac, Homocys-PL-Nac, Farcys-PL-Ole, Met-AG-PL, Pal-PL-Ole, Myr-PL-Ole, Tau-AG-PL, VitC-PL, Tau-G-PL, Stea-PL-Farcys, ACh-AG-PL, αtoco-PL, Aze-PL-Ole und andererseits die folgenden Konjugate (1b): Farcys-PL-Pal, Farcys-PL-Myr, Pal-PL-Myr, Aze-PL-Ole, Pal-PL-Ole, Farcys- -PL-Ole, PO-PL-Aze, Ole-PL-Myr, Stea-PL-Farcys, Ole-PL-Laur enthält;
* einer Assoziation, die in zwei unterschiedlichen Packungen einerseits die folgenden Konjugate (2a): Myr-PL-Pal, Pal-PL-Ole, Myr-PL-Ole, Tau-AS-PL oder Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL oder Met-AG-PL, Homocys-AS-PL oder Homocys-AG-PL, Cys-G-PL, Homocys-G-PL, Met-G-PL, CHO-PL, 5MT-AG-PL, W-AG-PL, ACh-AG-PL und andererseits die folgenden Konjugate (2b): Pal-PL-Ole, Tau-AS-PL oder Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL oder Met-AG-PL, Homocys-AS-PL oder Homocys-AG-PL, Cys-AG-PL, Cys-G-PL, Met-G-PL, Homocys-G-PL, Met-G-PL, CHO-PL, W-AG-PL, ACh-AG-PL, AI enthält;
* einer Assoziation, die in drei unterschiedlichen Packungen
3a) die folgenden Konjugate: Myr-PL-Pal, Pal-PL-Ole, Myr-PL-Ole, Tau-AS-PL oder Tau-AG-PL, αtoco-PL, VitC-PL, Homocys-PL-Nac, Cys-PL-Nac, Met-PL-Nac, Farcys-PL-Myr, Farcys-PL-Pal, Farcys-PL-Ole, Farcys-PL-Stea, Met-AS-PL oder Met-AG-PL, Homocys-AS-PL oder Homocys-AG-PL, Cys-G-PL, Homocys-G-PL, Met-G-PL, CHO-PL, 5MT-AG-PL, W-AG-PL, ACh-AG-PL,
3b) die folgenden Konjugate: Cys-AG-PL, Met-AG-PL, Homocys-G-PL, Cys-PL-Nac, Homocys-PL-Nac, Met-PL-Nac, Tau-AG-PL, Tau-G-PL, Met-G-PL, αtoco-PL, αtoco-PL-Ret und
3c) die folgenden Konjugate: Farcys-PL-Ole, Aze-PL-Ole, Aze-PL-PO, Met-PL-Nac, Cys-PL-Nac, Homocys-PL-Nac, αtoco-PL, Lin2-PL-Ole, Ret-PL, Ole-PL-Laur, αtoco-PL-Ret, Tau-G-PL, ACh-AG-PL, PRO-PL, GABA-G-PL enthält;
* einer Assoziation, die in vier unterschiedlichen Packungen
4a) die folgenden Konjugate: Farcys-PL-Ole, PO-PL-Ole, Aze-PL-Ole, Aze-PL-PO, Met-PL-Nac, Homocys-PL-Nac, αtoco-PL, VitC-PL, Tau-AG-PL, Lin2-PL-Ole, CHO-PL, Ole-PL-Laur, 5-MT-AG-PL, Tau-G-PL, ACh-AG-PL;
4b) die folgenden Konjugate: Farcys-PL-Pal, Farcys-PL-Myr, Pal-PL-Myr, Aze-PL-Ole, Pal-PL-Ole, Farcys-PL-Ole, PO-PL-Aze, Ole-PL-Myr, Stea-PL-Farcys, Ole-PL-Laur;
4c) die folgenden Konjugate: Farcys-PL-Ole, Aze-PL-Ole, Aze-PL-PO, Met-PL-Nac, Cys-PL-Nac, Homocys-PL-Nac, αtoco-PL, Lin2-PL-Ole, Ret-PL, Ole-PL-Laur, αtoco-PL-Ret, Tau-G-PL, ACh-AG-PL, PRO-PL, GABA-G-PL und
4d) die folgenden Konjugate: Farcys-PL-Ole, Aze-PL-Ole, Aze-PL-PO, αtoco-PL, VitC-PL, Tau-AG-PL, Lin2-PL-Ole, CHO-PL, Laur-PL-Ole, AI enthält;
* einer Assoziation, die in drei unterschiedlichen Packungen
5a) die folgenden Konjugate: Farcys-PL-Pal, Pal-PL-Myr, Farcys-PL-Myr, Lin2-PL-Ole, Met-PL-Nac, Cys-PL-Nac, Homocys-PL-Nac, Farcys-PL-Ole, Met-AG-PL, Pal-PL-Ole, Myr-PL-Ole, Tau-AG-PL, VitC-PL, Tau-G-PL, Stea-PL-Farcys, ACh-AG-PL, αtoco-PL, Aze-PL-Ole;
5b) die folgenden Konjugate: Farcys-PL-Pal, Farcys-PL-Myr, Pal-PL-Myr, Aze-PL-Ole, Pal-PL-Ole, Farcys-PL-Ole, PO-PL-Aze, Ole-PL-Myr, Stea-PL-Farcys, Ole-PL-Laur; und
5c) die folgenden Konjugate: Farcys-PL-Pal, Pal-PL-Myr, Farcys-PL-Myr, Farcys-PL-Ole, Myr-PL-Ole, Stea-PL-Farcys, ACh-AG-PL, αtoco-PL, AI enthält;
* einer Assoziation, die in zwei unterschiedlichen Packungen einerseits die folgenden Konjugate (6a): Lin2-PL-Farcys, Stea-PL-Farcys, Myr-PL-Farcys, Pal-PL-Farcys, Ole-PL-Farcys, Cys-AG-PL, Met-AG-PL, Homocys-AG-PL, CHO-PL, Homocys-PL-Nac, Cys-PL-Nac, αtoco-PL-Ret, αtoco-PL, VitC-PL, Ole-PL-Laur, Met-G-PL, Met-PL-Nac, Homocys-PL-Nac, Cys-G-PL
und andererseits die unter obigem Punkt 3c) genannten Konjugate enthält, und
* einer Assoziation, die in drei unterschiedlichen Packungen
7a) die folgenden Konjugate: PRO-PL, Ret-PL, αtoco-PL, αtoco-PL-Ret, W-AG-PL, 5MT-AG-PL, Hist-PL;
7b) die unter obigem Punkt 1b) genannten Konjugate und
7c) die folgenden Konjugate: L-DOPA-G-PL, L-DOPA-AG-PL, 5MT-AG-PL, HW-AG-PL, Tyr-AG-PL enthält.

14. Nahrungsmittel- oder Vitaminzusatz, der mindestens ein Konjugat wie in einem der Ansprüche 7 bis 9 definiert enthält.
